(19) Europäisches Patentamt

European Patent Office

Office européen des brevets

(11) **EP 4 751 669 A2**

(12) **EUROPEAN PATENT APPLICATION**

(43) Date of publication:
**03.06.2026 Bulletin 2026/23**

(21) Application number: **26157233.3**

(22) Date of filing: **30.12.2020**

(51) International Patent Classification (IPC):
***A61B 18/04*** *(2006.01)*

(52) Cooperative Patent Classification (CPC):
**A61B 5/0538; A61B 1/307; A61B 5/4839;**
**A61B 17/3478; A61B 18/04;** A61B 1/00066;
A61B 2017/00274; A61B 2018/00547;
A61B 2018/00875; A61B 2018/048;
A61B 2034/2051; A61B 2090/064; A61B 2090/065;
A61B 2090/3782; A61B 2090/3784

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **30.12.2019 US 201962955288 P**

(62) Document number(s) of the earlier application(s) in
accordance with Art. 76 EPC:
**20910200.3 / 4 084 668**

(71) Applicant: **Francis Medical, Inc.**
**Maple Grove, MN 55369 (US)**

(72) Inventors:
• **HOEY, Michael**
**Maple Grove, 55369 (US)**

• **HASTINGS, Roger Noel**
**Maple Grove, 55369 (US)**

(74) Representative: **J A Kemp LLP**
**80 Turnmill Street**
**London EC1M 5QU (GB)**

Remarks:
•This application was filed on 09.02.2026 as a
divisional application to the application mentioned
under INID code 62.
•Claims filed after the date of filing of the application /
after the date of receipt of the dvisional application
(Rule 68(4) EPC).

(54) **VAPOR THERAPY SYSTEMS AND METHODS**

(57) A vapor delivery system is provided that may include any of a number of features. One feature of the vapor delivery system is that it can apply condensable vapor energy to tissue, such as a prostrate, to shrink, damage, or denature the prostate. In some embodiments, the vapor delivery system can include safety features including prostate capsule detection, needle tracking, and treatment tracking. Methods for safe and effective treatment of prostate tissues are presented.

FIG. 1A

EP 4 751 669 A2

**Description**

**CROSS-REFERENCE TO RELATED APPLICATIONS**

This application claims the benefit of priority of U.S. Provisional Application No. 62/955,288, filed December 30, 2019, herein incorporated by reference in its entirety.

**INCORPORATION BY REFERENCE**

All publications, including patents and patent applications, mentioned in this specification are herein incorporated by reference in their entirety to the same extent as if each individual publication was specifically and individually indicated to be incorporated by reference.

**FIELD**

The present invention relates to devices and related methods for treatment of prostate cancer using a minimally invasive approach.

**BACKGROUND**

The human male prostate can be classified into three zones: the peripheral zone, transition zone, and central zone. Peripheral zone (PZ) comprises about 70% of the volume of a male's prostate. This sub-capsular portion of the posterior aspect of the prostate gland surrounds the distal urethra and 70 to 80% of cancers originate in the peripheral zone tissue. The central zone (CZ) surrounds the ejaculatory ducts and contains about 20-25% of the prostate volume. The central zone is often the site of inflammatory processes. The transition zone (TZ) is the site in which benign prostatic hyperplasia (BPH) develops and contains about 5-10% of the volume of glandular elements in a normal prostate, but can constitute up to 80% of such volume in cases of BPH. The transition zone includes two lateral prostate lobes and the periurethral gland region. There exist natural barriers around the transition zone, i.e., the prostatic urethra, the anterior fibromuscular stroma (FS), and a fibrous plane (FP) between the transition zone and peripheral zone. The anterior fibromuscular stroma (FS) or fibromuscular zone is predominantly fibromuscular tissue.

Approximately 70% to 80% of prostate cancers originate in the peripheral zone of the prostate and may be confined to the peripheral zone. In recent years, there has been an increased interest in focal therapy for prostate cancer, treating only regions of tissue in which cancer has been found following biopsies. Prior art focal therapy treatments, such as with RF ablation energy, may not confine the treatment to the peripheral zone tissue or to tissues within the prostate.

**SUMMARY OF THE DISCLOSURE**

A prostate treatment system is provided, comprising an introducer shaft sized and configured for transurethral access into a patient, a handle coupled to the introducer shaft, a vapor generator disposed in the handle and configured to generate a condensable vapor, a vapor delivery needle in communication with the vapor generator and slidably disposed within the introducer shaft, a magnet attached to the needle, a solenoid actuator disposed around the magnet, the solenoid actuator providing controlled movements of the needle to deploy into tissue, advance at constant speed or in pulsed steps, and retract into the shaft, sensors disposed on the needle, shaft, and handle to provide data on the needle tip location and heading within tissue, and electronics disposed in the handle and an external console that communicate with each other and with the user to insure safe and effective delivery of vapor to targeted tissues and to prevent delivery of vapor outside of targeted tissues.

A prostate treatment device is provided, comprising an introducer shaft sized and configured for transurethral access into a patient, a therapy needle slidably disposed within the introducer shaft, an advancement mechanism coupled to the therapy needle and configured to advance the therapy needle from the introducer shaft through a prostatic urethra into a prostate of the patient, at least one sensor disposed on a distal portion of the therapy needle, the at least one sensor being configured to sense a parameter of one or more tissues of the prostate, and an electronic controller operatively coupled to the at least one sensor, the electronic controller being configured to determine if the therapy needle contacts a prostatic capsule of the prostate based on the sensed parameter.

In some embodiments, the parameter comprises an electrical impedance of one or more tissues of the prostate.

In one embodiment, sensing the electrical impedance of the one or more tissues of the prostate further comprises passing a constant current amplitude sine wave at a fixed frequency to the at least one sensor, measuring a voltage amplitude of the at least one sensor, determining an impedance amplitude by calculating a ratio of the voltage amplitude and the current amplitude, determining a phase shift between the voltage amplitude and the current amplitude, and

computing the electrical impedance of the one or more tissues with the phase shift and the impedance amplitude.

In one embodiment, the at least one sensor comprises at least one bio-impedance electrode.

In some embodiments, the controller is configured to determine that the therapy needle has contacted a prostatic capsule of the prostate when there is an abrupt change in the electrical impedance.

In one embodiment, the abrupt change comprises a sudden change of more than 25%.

In some examples, the parameter comprises an electrical resistance of one or more tissues of the prostate. In another example, the parameter comprises an electrical capacitance of one or more tissues of the prostate. In yet another example, the parameter comprises a force applied by one or more tissues of the prostate to the at least one sensor.

In some embodiments, the at least one sensor comprises a force sensor. In one example, the force sensor is embedded behind a flexible tip of the therapy needle, wherein the flexible tip is configured to flex when a critical force is applied to the flexible tip.

In some embodiments, the therapy needle is configured to deliver vapor into the prostate.

In one example, the device further comprises a magnet coupled to a proximal portion of the therapy needle, wherein the advancement mechanism comprises a solenoid actuator disposed around the magnet, the solenoid actuator comprising a push winding coupled to a source of current and a pull winding coupled to the source of current, the push winding being configured to apply a first magnetic field to the magnet, the pull winding being configured to apply a second magnetic field to the magnet, wherein the first and second magnetic fields move a distal tip of the therapy needle between a retracted position inside the introducer shaft and an extended position at least partially outside of the introducer shaft.

A method of treating a prostate of a patient is also provided, comprising the steps of inserting a shaft of a therapy device transurethrally into the patient, advancing a therapy needle from the shaft, through a prostatic urethra of the patient, and into the prostate of the patient, measuring at least one parameter of prostate tissue with a sensor disposed on the therapy needle, determining that the therapy needle has contacted a prostatic capsule based on the at least one parameter, and stopping advancing the therapy needle when the prostatic capsule is contacted.

In some embodiments, the at least one parameter comprises an electrical impedance of the prostate tissue.

In another embodiment, determining that the therapy needle has contacted the prostatic capsule further comprises detecting an abrupt change in the measured electrical impedance. In some embodiments, the abrupt change comprises a sudden change of more than 25% in the measured electrical impedance.

In one embodiment, the at least one parameter comprises a force applied by the prostate tissue to the therapy needle.

In some examples, determining that the therapy needle has contacted the prostatic capsule further comprises detecting a critical force with the sensor.

In some embodiments, the method further comprises delivering vapor from the therapy needle into the prostate.

A prostate treatment system is provided, comprising a therapy device comprising an introducer shaft sized and configured for transurethral access into a patient, a therapy needle slidably disposed within the introducer shaft, an advancement mechanism coupled to the therapy needle and configured to advance the therapy needle from the introducer shaft through a prostatic urethra into a prostate of the patient, at least one transmitter disposed on the therapy needle, and an external tracking system configured to sense a position of the at least one transmitter within the prostate.

In some embodiments, the at least one transmitter comprises a magnet, wherein the external tracking system is configured to sense a pulsed magnetic field from the magnet to determine the position of the at least one sensor within the prostate.

In another embodiment, the external tracking system comprises an array of transmitter coils configured to sense a change in ambient magnetic field of the magnet as it moves through the prostate.

In some embodiments, the external tracking system is disposed on or within a trans-rectal probe.

In another embodiment, the trans-rectal probe comprises a trans-rectal ultrasound probe.

In some embodiments, the magnet comprises an electromagnet.

A prostate treatment device is provided, comprising an introducer shaft sized and configured for transurethral access into a patient, a therapy needle slidably disposed within the introducer shaft, an advancement mechanism coupled to the therapy needle and configured to advance the therapy needle from the introducer shaft through a prostatic urethra into a prostate of the patient, at least one transmitter disposed on the therapy needle, and a tracking sensor disposed on a distal portion of the introducer shaft, the tracking sensor being configured to sense a position of the at least one transmitter on the therapy needle relative to the distal portion of the introducer shaft.

In some embodiments, the at least one transmitter comprises a magnet, wherein the tracking sensor is configured to sense a pulsed magnetic field from the magnet to determine the position of the at least one sensor.

In another embodiment, the external tracking system comprises an array of transmitter coils configured to sense a change in ambient magnetic field of the magnet as it moves through the prostate.

In some embodiments, the magnet comprises an electromagnet.

A method of treating a prostate of a patient is provided, comprising the steps of inserting a shaft of a therapy device transurethrally into the patient, advancing a therapy needle from the shaft, through a prostatic urethra of the patient, and into the prostate of the patient, determining a real-time position of the therapy needle in the prostate, displaying the real-time position of the therapy needle and the prostate, and providing ablative therapy from the therapy needle to the prostate.

In some embodiments, the advancing step further comprises advancing the therapy needle and a transmitter disposed on the therapy needle into the prostate.

In other embodiments, determining the real-time position of the therapy needle further comprises sensing an ambient magnetic field of the transmitter with a tracking system.

In some embodiments, the method further comprises sensing the ambient magnetic field of the transmitter with a tracking system disposed on the shaft of the therapy device.

In some embodiments, the method further comprises sensing the ambient magnetic field of the transmitter with a tracking system external to the therapy device.

In some embodiments, the method further comprises sensing the ambient magnetic field of the transmitter with a tracking system disposed on a trans-rectal probe.

In some embodiments, the method further comprises sensing the ambient magnetic field of the transmitter with a tracking system disposed on a trans-rectal ultrasound probe.

In some embodiments, the method further comprises registering the real-time position of the therapy needle onto an ultrasound image from the trans-rectal ultrasound probe.

In another embodiment, the method includes displaying the real-time position further comprises displaying the real-time position of the therapy needle within the prostate.

A method for tracking prostate therapy of a patient is provided, comprising the steps of generating vapor in a vapor therapy system, delivering vapor from the vapor therapy system into a first location within a prostate of the patient, injecting a first volume of air from the vapor therapy system into the first location, and visualizing the first volume of air in the prostate to track the prostate therapy.

In one embodiment, delivering vapor further comprises introducing a shaft of the vapor therapy system transurethrally into the patient, advancing a vapor therapy needle from the shaft into the first location of the prostate.

In one example, the method further comprises delivering vapor from the vapor therapy system into a second location within the prostate, injecting a second volume of air from the vapor therapy system into the second location, and visualizing the second volume of air in the prostate to track the prostate therapy.

In one embodiment, the first volume of air is larger than the second volume of air.

In another embodiment, the first volume of air is smaller than the second volume of air.

In other embodiments, the method further comprises creating a map of treated prostate locations.

In some embodiments, injecting the first volume of air is performed after delivering vapor into the first location. In other embodiments, injecting the first volume of air and delivering vapor into the first location are performed simultaneously.

## BRIEF DESCRIPTION OF THE DRAWINGS

In order to better understand the invention and to see how it may be carried out in practice, some preferred embodiments are next described, by way of non-limiting examples only, with reference to the accompanying drawings, in which like reference characters denote corresponding features consistently throughout similar embodiments in the attached drawings.

FIGS. 1A-1E show one embodiment of a vapor delivery system.

FIGS. 2A-2C illustrate one embodiment of a TRUS probe for use with the vapor delivery system to provide real-time tracking of the vapor delivery needle tip.

FIGS. 3A-3B illustrates the infusion of air with vapor to track the zone of vapor ablation.

FIGS. 4A-4C illustrate one embodiment of tracking a position of the vapor delivery needle with a needle tip magnet or coil.

FIGS. 5A-5C show a vapor delivery needle with a tip coil sensor for motion tracking.

FIGS. 6A-6E illustrate other embodiments of tracking a position of the vapor delivery needle with trans-rectal probes.

FIGS. 7-8 illustrate further embodiments of a vapor delivery needle with contact sensors configured to detect when the needle tip contacts the prostate wall.

FIGS. 9A-9B are an example of a vapor delivery needle with a non-Newtonian tip configured to deform when making contact with a prostatic capsule.

FIG. 10 is a flowchart describing another method of treating prostate tissue.

**DETAILED DESCRIPTION**

In general, one method for treating cancer of the prostate comprises introducing a heated vapor interstitially into the interior of a prostate, wherein the vapor controllably ablates prostate tissue. This method can utilize vapor for applied thermal energy of between 50 calories and 600 calories per each individual vapor treatment (and assumes multiple treatments for each prostate lobe) in an outpatient-based procedure. The method can cause localized ablation of prostate tissue without damaging the prostatic urethra and without damaging tissue outside of the prostate gland.

The present disclosure is directed to the treatment of prostate cancer, and more particularly for ablating peripheral zone prostate tissue without ablating central or transitional zone prostate tissue.

The system can include a vapor delivery mechanism that delivers vapor media, including water vapor. The system can utilize a vapor source configured to provide vapor having a temperature of at least 60°-140° C. In another embodiment, the system further comprises a computer controller configured to deliver vapor for an interval ranging from 1 second to 30 seconds.

In some embodiments, the system further comprises a source of a pharmacologic agent or other chemical agent or compound for delivery with the vapor. These agents include, without limitation, an anesthetic, an antibiotic or a toxin such as Botox®, or a chemical agent that can treat cancerous tissue cells. The agent also can be a sealant, an adhesive, a glue, a superglue or the like.

In some embodiments, a prostate treatment device can be provided comprising an introducer shaft sized and configured for transurethral access into a patient, a vapor generator configured to generate a condensable vapor, a vapor delivery needle in communication with the vapor generator and slidably disposed within the introducer shaft, and an actuator configured to move the vapor delivery needle between a retracted position inside the introducer shaft and an extended position at least partially outside of the introducer shaft, and to advance or retract the needle continuously or in steps to tissues at any location between the prostatic urethra and prostate capsule.

This disclosure is directed to safe and effective delivery of vapor to ablate tissue. A vapor delivery device can include a shaft configured for transurethral access to a patient's prostate, a vapor generator, and a vapor delivery needle that can include one or more vapor delivery ports. In one embodiment vapor is delivered through the port(s) of the vapor delivery needle to ablate cancerous or precancerous tissue. In a preferred embodiment, the vapor delivery needle is configured to puncture the prostatic urethra and advance to one or more sites within the prostate where vapor is delivered. Multiple puncture sites can be spaced apart to provide overlapping zones of tissue ablation in the prostate, without being close enough together to allow vapor delivered at a site to exit through the entry holes of the previous puncture sites.

More specifically, this disclosure is directed to navigation of a vapor delivery device, including a vapor delivery needle, into and throughout the prostate without the possibility of penetrating the prostate capsule. In some embodiments, the vapor delivery needle tip is blunted to a degree that it penetrates the prostatic urethra under large deployment forces, yet it cannot puncture the prostate capsule under smaller navigation forces. In other embodiments electrodes are disposed on the needle tip to measure tissue electrical impedance adjacent the tip. Tissue impedance, (both resistance and capacitance) change abruptly as the tissue changes from cellular within the prostate to fibrous in the capsule wall.

Systems and methods are disclosed for sensing the force exerted by tissue on the vapor delivery needle tip and alerting the user when a force large enough to puncture the prostate capsule is approached. In one embodiment, linear force or displacement sensors may be disposed on the vapor delivery device, including on the vapor delivery needle. Alternatively, the system may include a switch closure feature that activates when a specified tip force has been exceeded.

In another embodiment, the needle is advanced by applying a force to the needle via an electronic solenoid to which the needle is attached. Sensors adjacent the solenoid measure the displacement of the solenoid magnet and needle. Using a closed loop algorithm, the needle is advanced a specified distance or at a specified speed. If the needle encounters an obstruction, the solenoid current is automatically increased to continue needle movement. A critical current corresponds to a critical force on the needle that is equal and opposite to the force exerted on the needle by prostate tissue. A critical force may be defined indicating that the needle has encountered the prostate capsule. At this point the solenoid force may be taken to zero and the operator may be warned that a critical obstruction has been encountered which may be the wall of the prostate capsule.

In another embodiment, the needle tip can be constructed from a non-Newtonian material that maintains a needle tip shape when advanced rapidly during needle deployment to puncture the urethra, but is deformed to a blunt tip when smaller, slowly acting forces are applied. The blunt tip shape cannot penetrate the prostate capsule.

In a preferred embodiment, the vapor delivery needle tip is blunted to a degree that penetration of the prostate capsule cannot occur under the forces applied by the needle driver during navigation of prostate tissues. On the other hand, the much higher needle speed achieved during initial needle deployment is adequate for the blunt tip to penetrate the urethra wall. In one embodiment, the needle driver comprises a solenoid. Initial deployment of the needle can occur in 10 to 15 msec, and the needle may reach speeds in excess of 1 m/sec, adequate for blunted needle tips to penetrate the urethral wall. During navigation through prostate tissue, the solenoid may be activated for times of about 1 to 2 msec, adequate for the needle to travel a small, specified distance. Due to frictional forces, the needle does not reach its terminal speed during the short pulse duration, and the needle reaches speeds that are less than 1 m/sec, or less than 0.5 m/sec. In one embodiment, the needle is advanced in a continuous movement through prostate tissue, which requires solenoid forces that are much smaller than the initial deploy force. The force is measured and controlled via an algorithm to prevent forces high enough to penetrate the capsule wall.

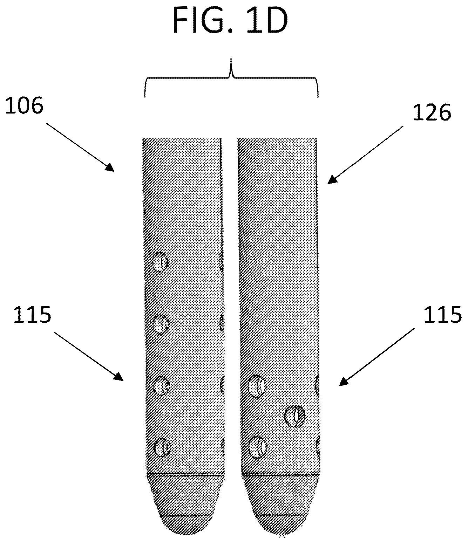

Experiments that we have performed on human and simulated prostate tissues suggest that hemispherical tips having a diameter in the range of 0.020 inches to 0.035 inches will indeed penetrate the urethra wall without penetrating the prostate capsule. In this example, the needle diameter of 0.050 inches tapers at an angle of about 17 degrees down to a hemispherical tip having diameters in the range of 0.20 inches to 0.35 inches. The most preferred diameter in our experimentation was 0.032 inches. Needle tip blunting is a simple, passive means for preventing breach of the prostate capsule during vapor therapy. Needles shown in Figure 1D are examples of blunted tips.

**Vapor Delivery System**

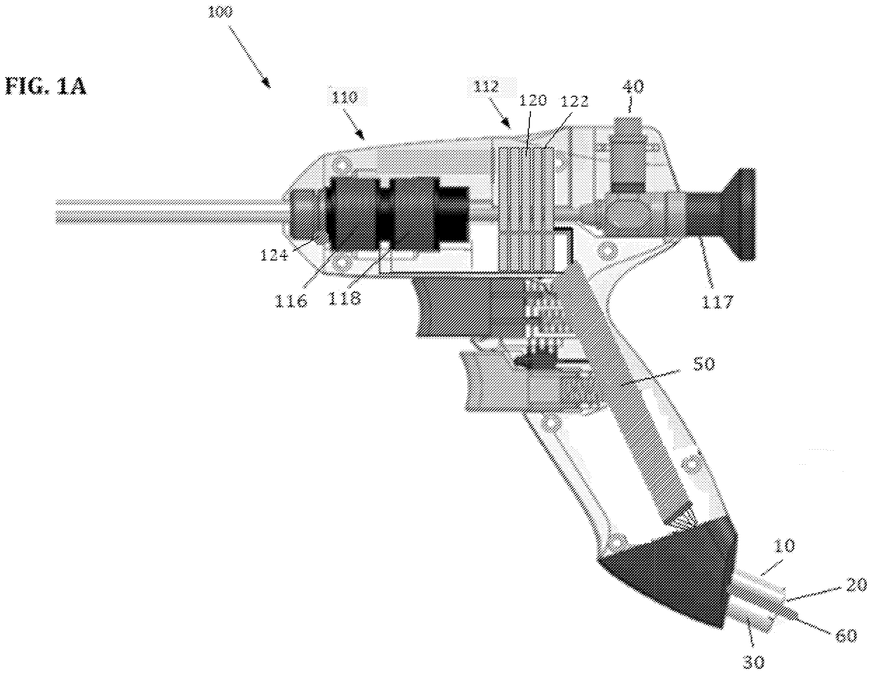

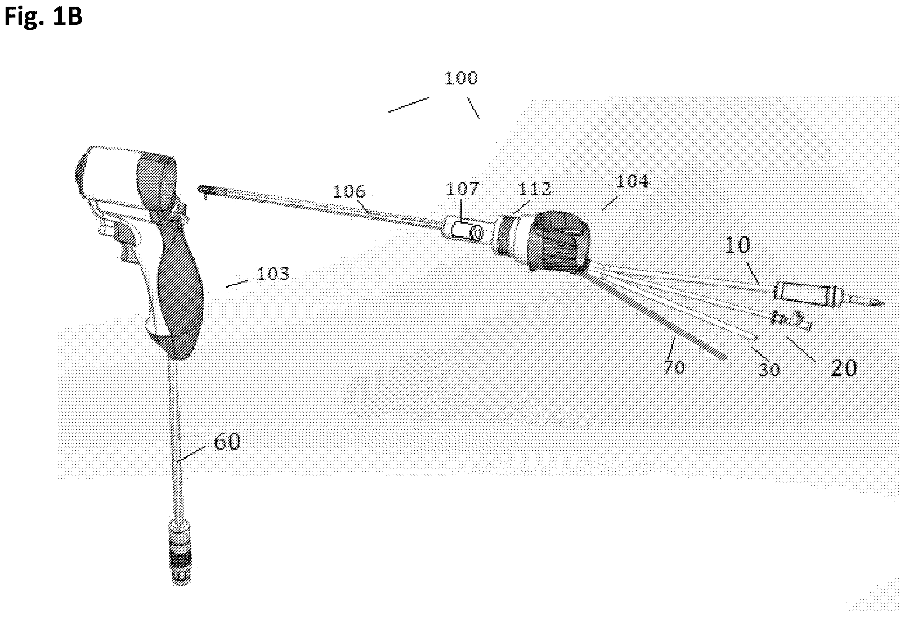

FIG. 1A shows one embodiment of a vapor delivery system 100. Vapor delivery system 100 can have an elongate shaft 102 configured for insertion into the urethra of a patient and a handle portion 104 for gripping with a human hand. The handle can be an ergonomic swept back handle that allows the user to comfortably rotate the delivery device left and right to deliver vapor to the right and left lobes of the prostate. The vapor delivery system 100 can include a vapor delivery needle 106 of FIG. 1B disposed in the shaft and configured to extend from a distal portion of the elongate shaft 102.

The vapor delivery system 100 can further include one or more triggers, buttons, levers, or actuation mechanisms configured to actuate the various functions of the system, including delivery of vapor, needle advancement/retraction, saline or cooling fluid flush, etc. In some embodiments advancement of the needle can be visualized through a cystoscope 117 via markings along the distal length of the needle. In other embodiments one or more magnetic sensors detect the magnetic field of magnet 107 that drives the vapor delivery needle. Electronics convert the magnetic field measurement to magnet (and needle) position which may be displayed on a monitor.

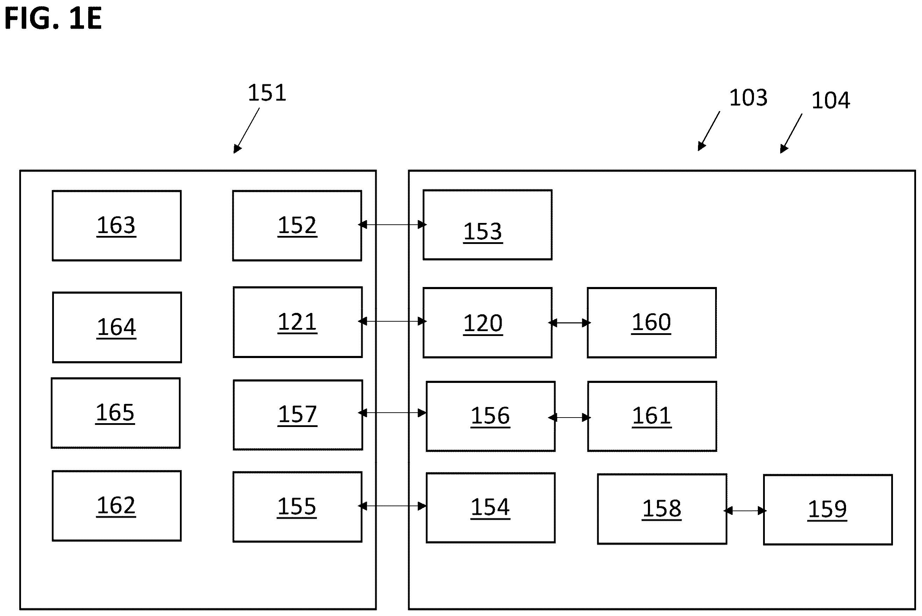

The vapor delivery system 100 can comprise a sterile water source 10, an aspiration source 20, a fluid cooling or irrigation source 30, a light source 40, an electronic controller 50 configured to control generation and delivery of vapor from the vapor source, through a lumen of the shaft, through the vapor delivery needle, and into tissue. In some embodiments, the electronic controller can be disposed on or in the vapor delivery system, and in other embodiments the electronic controller can be disposed in the external console of FIG. 1E. In a preferred embodiment, the controller electronics reside partly within the delivery system and partly within the external console, and the two electronics boards are connected by cable 60.

The vapor delivery system 100 of FIGS. 1A-1E can further include a solenoid needle driver 110 and vapor generator 112. The solenoid needle driver 110 can be configured to advance and retract the vapor delivery needle 106 of the vapor delivery system. The solenoid needle driver can include the vapor delivery needle, a pull winding 116, a push winding 118, and a magnet 107 disposed on the vapor delivery needle. The magnet 107, and thus the vapor delivery needle, can then be moved laterally by generating magnetic fields in the push and pull windings 116 and 118 to advance and retract the vapor delivery needle. During retraction the current through the solenoid is reversed, and coil 116 becomes the push winding and coil 118 becomes the pull winding. The solenoid current may be controlled in real time and in response to one or more sensors 124 that detect the position of the needle driver magnet. In a preferred embodiment one or more Hall magnetic field sensors are used to measure the magnetic field of magnet 107 and thereby compute its location relative to the retracted position. In some embodiments the solenoid current is measured and used to compensate the Hall sensor signals for the magnetic field created by current in the solenoid coils. Closed loop control of the needle magnet location is achieved in a PID control loop in which the difference between commanded and actual magnet locations is driven to zero by continuous adjustment of the solenoid current. In closed loop control modes, pulsed and continuous needle movement can thereby be achieved at variable rates and at known and carefully controlled magnet and needle locations relative to the retracted location. In some embodiments the needle can be commanded to move at a rate of 0.5 mm/sec, or in a range of speeds between 0.1 mm/sec and 10 mm/sec, or in a range of speeds between 0.25 mm/sec and 4 mm/sec. Since the speed is maintained by the control loop, the solenoid current will automatically increase when the needle tip encounters an obstruction, for example the prostate capsule wall. As a safety feature, the solenoid current may be monitored in real time and an alert or automatic shutdown performed when the solenoid current exceeds a critical value indicating the presence of an obstruction, especially important if the obstruction is the capsule wall.

The vapor generator 112 can comprise a coiled metal tube 120 through which sterile water flows that is converted to high quality vapor when DC or AC current is passed through the walls of the tube. The vapor passes through the delivery device needle for delivery to targeted tissues. The vapor generator 112 can include a coiled tube 120 of Inconel 625 stainless steel with electrical leads for DC or AC current attached at its ends. The Inconel tube is covered by a thin wall, electrical insulating material 122, such as polyimide, to ensure current flow through the entire length of the tube without shorting between adjacent tube windings. Inconel 625 is an example of a metal that has a very small temperature coefficient of electrical resistance, so that Ohmic heat is applied uniformly along the length of the tube regardless of a substantial temperature gradient along its length. Polyimide is an excellent electrical insulator across a thickness small enough to provide good thermal conductivity between adjacent tube windings, thereby minimizing the temperature gradient along the heating element tube. The polyimide insulation may have a thickness between 10 and 100 microns. Sterile water can be introduced into the vapor generator coil 120, and DC or AC current applied through the walls of the vapor generator coil tube to dissipate Ohmic heat in the electrical resistance of the tube material along the tube length. The vapor coil can be connected to a supply of sterile water through a plastic tube 10 that extends from the inner coil to a fluid source. Vapor exits the distal end of the tube into a vapor delivery needle 106 made, for example, from PEEK. Consistent caloric vapor output is ensured by measuring and controlling the Ohmic heating power dissipated the tube. Heating power is measured as the product of current flowing through the tube and voltage across the tube. Heating power is controlled in real time to a set value.

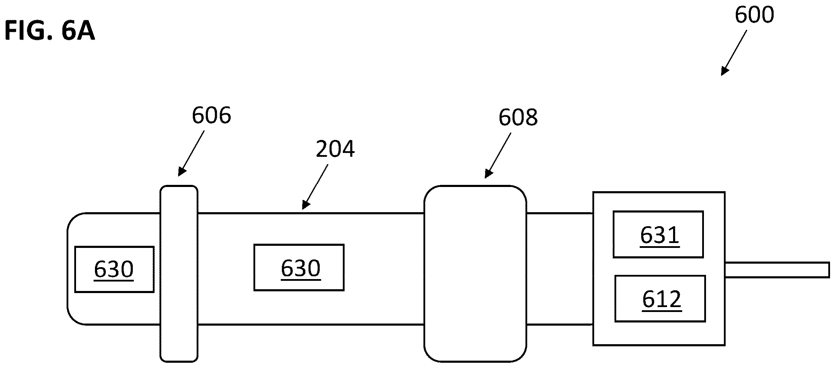

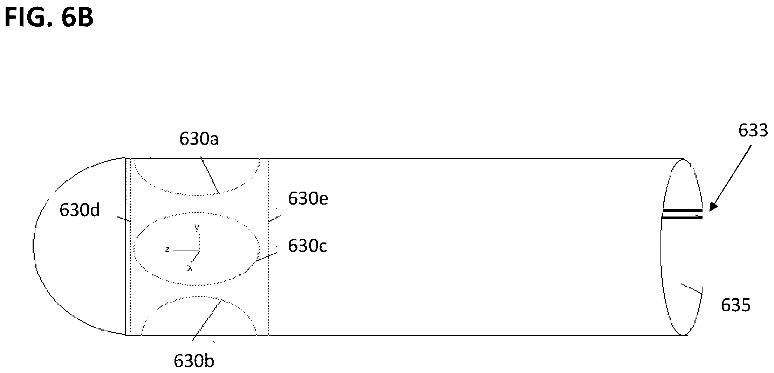

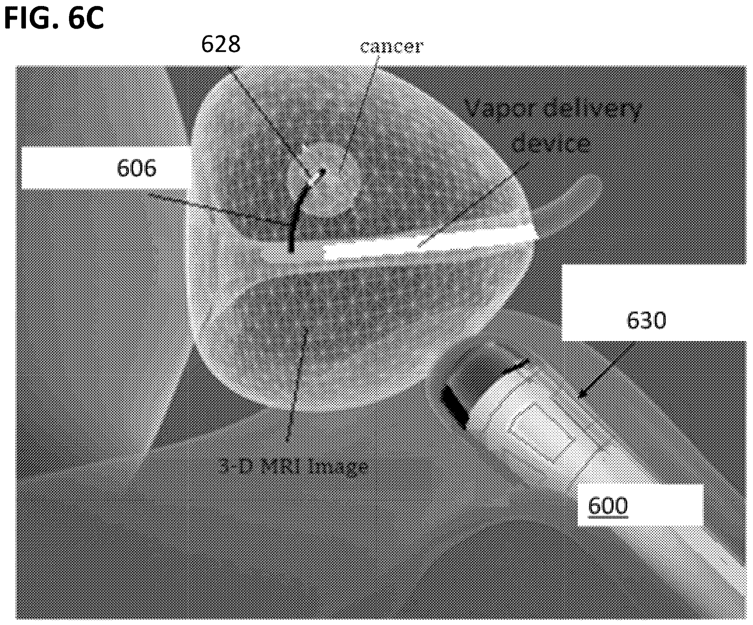

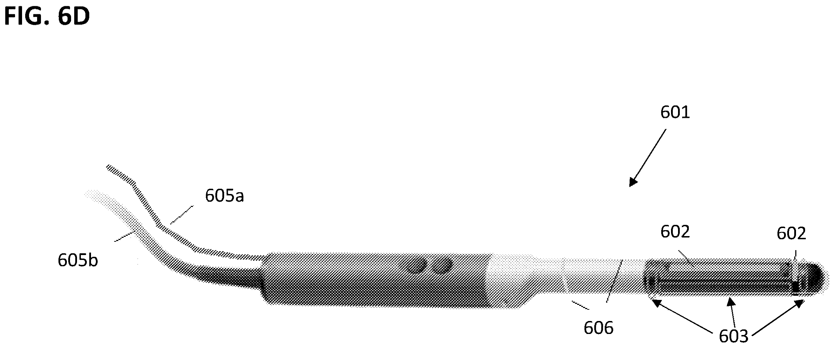

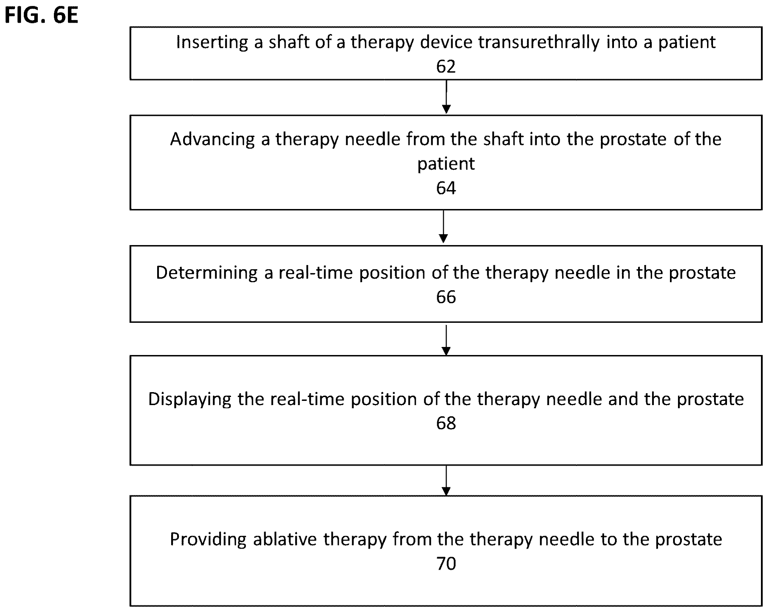

FIG. 1B shows one embodiment of a vapor delivery system 100 which comprises two separable systems, the delivery device handle 103, and cartridge 104 which may be removed from handle 103. The handle 103 comprises buttons and actuators that engage circuit board 50 that, along with electronics in the external console, control needle movements and the delivery of vapor, and process and/or relay information from sensors such as thermocouples on the heating element 112 (not shown) and sensors disposed on needle 106 (for example in FIG. 6) that sense needle location and orientation relative to prostate tissues. Cable 60 connects sensors and electronics within the delivery device handle 103 with an external control system. Cable 70 connects the cartridge heating element 112 and sensors to the external console. Alternatively, cable 70 may plug into delivery device handle 103, with some of the leads passing through cable 60 to the console. Cables 60 and 70 from handle 103 and cartridge 104 may join at a point lying between the delivery system and the external console, entering the external console via a single connector.

In one embodiment, handle 103 is reusable while cartridge 104 is disposable. In general, the two-piece design allows cartridges to be inserted for different aspects of a procedure. For example, in a prostate ablation procedure, a cartridge containing needle 126 of FIG. 1D may be used to treat peripheral zones of the prostate while a cartridge containing needle 106 is used for treatment of other prostate zones. Cartridges may contain needles having other hole patterns and various needle lengths for treating other tissues. Some cartridges may contain sophisticated sensor systems, e.g., for tracking the needle tip or measuring vapor temperature or pressure at the needle tip. Cartridges may have a range of features and costs. The two-piece design of FIG. 1B allows application specific cartridges to be exchanged without throwing away the handle containing relatively expensive electronics.

In the two-piece design of FIG. 1B, cartridge 104 can rotate within handle 103. This feature allows the handle 103 to be held stationary while rotating the cartridge and needle tip to penetrate prostate tissues surrounding the prostatic urethra. The single piece system of FIG. 1A requires the user to rotate the entire device to address all prostate tissue.

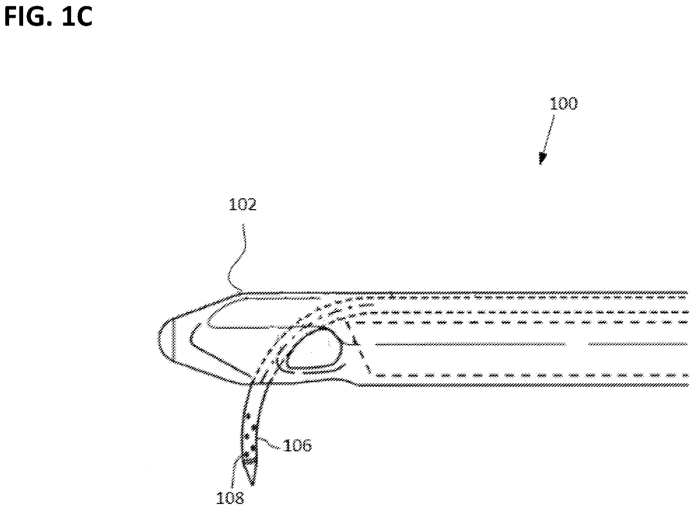

FIG. 1C shows a close-up view of the distal portion of the shaft of vapor system 100, including the vapor delivery needle 106 extending beyond the shaft and exposing the vapor delivery ports 108. The vapor delivery needle can extend generally perpendicular to or transverse from the shaft. The needle tip can include one or more vapor delivery ports 108 configured to deliver a flow of vapor media from the needle into prostate tissue. Vapor delivery ports 108 may be arranged in a pattern that optimizes the delivery of vapor to tissue in a given application. In general, the vapor delivery ports can each have a unique diameter. In one embodiment the vapor delivery ports all have the same diameter. In one embodiment the vapor delivery holes occupy a reduced length near the needle tip for delivery of vapor to thin portions of prostate peripheral zones without vapor passing into adjacent prostate zones.

FIG. 1D compares a standard hole pattern of vapor delivery ports 115 in needle 106 comprising three rows of four vapor delivery ports spaced evenly around the needle circumference occupying 4 mm along the needle distal end. FIG. 1D also illustrates one example of a peripheral zone needle 126, comprising vapor delivery ports 115 spaced in a staggered pattern. In the illustrated example, needle 126 includes 9 vapor delivery ports occupying only 2 mm on the needle distal end. The diameter of the holes in the nine-hole patters is chosen so that the total cross-sectional area of all vapor holes remains the same as the 12-hole pattern, thereby ensuring that the vapor exit speed is approximately the same for both needles.

The system 100 can further include a lumen (lumen 117 in FIG. 1A) sized to accommodate an endoscope or camera to provide additional viewing and feedback to the physician. This endoscope or camera can provide a view of the distal end of the shaft and adjacent tissues, including a view of the vapor delivery needle when deployed, and markings on the needle that show needle deployment length.

FIG. 1E is one example of the various electronics components incorporated into the vapor delivery system described above. The system can include an electronic controller or external console 151, a handle 103, and a cartridge 104, as described above. For illustrative purposes, the cartridge and handle are schematically illustrated together, since in some embodiments these components are not separate, and additionally many of the electronics can be interchangeably placed in either the handle or cartridge of the system. The electronics are distributed between the console, cartridge, and delivery device handle in FIG. 1E, although it should be understood that the electronics could be moved between the various components. FPGA 152 disposed in the console and FPGA 153 disposed in the handle are configured to communicate with each other to monitor and process system sensors and actuators and control needle movements and vapor delivery. Advantages of the FPGAs include very high speed for real time feedback of rapidly changing signals, small size, and parallel processing architecture for simultaneously addressing multiple delivery system functions.

The vapor generator of the system can include a vapor coil 120 and a vapor controller 121 configured to control DC power delivery to the coil to heat the coil and produce vapor. In some embodiments, the vapor coil can be disposed within the handle of the system. Delivery of saline or fluid from the console to the generator can be achieved by controlling a saline pump with FPGA 152. Vapor generator current needed to deliver maximum vapor flow rates into tissue can range up to 25 Amps in some applications. Relatively heavy current carrying leads having low electrical resistance can comprise two parallel strands of AWG#22 magnet wire on both the go and return leads. These leads may pass through the delivery device handle or be routed to the console without entering the delivery device handle. Similarly, some or all of the leads from sensors in the delivery device cartridge may be routed through the handle for processing in FPGA 153, or they may be routed directly to the console for processing in FPGA 152.

The system can implement a solenoid 156 within the handle/cartridge to advance/retract the vapor delivery needle. A magnet on the needle can be moved by the solenoid, as described herein. Actuation of the vapor delivery needle for advancement, retraction, can be controlled by solenoid controller 157.

They system can include a plurality of sensors disposed in the console, handle, and/or cartridge. For example, sensors having leads that terminate in the cartridge include needle tip bio-impedance electrodes 154 configured for sensing proximity of the needle to the prostate capsule. Corresponding bio-impedance electronics 155 can be disposed in either the handle/cartridge or the console. Needle tip tracking electronics 158 and one or more coils 159 can be configured for sensing external magnetic fields used to track the location and heading of the needle tip (alternatively this coil may transmit an AC magnetic field that is sensed by external sensors). In some embodiments, the external magnetic fields are generated with tracking coils 162 of the console. These tracking coils can be positioned externally to the console, for example. In some embodiments, one or more thermocouples 160 located on the vapor generator coil 120 measure vapor outlet temperature and enable auto shut down of heating current when the temperature is out of range. Leads attached to the ends of the vapor generator can be configured to measure the voltage across the vapor generator for computation of power delivered to the vapor generator. Sensor leads may comprise fine insulated wire, for example AWG #30 magnet wire. These leads may be routed to the delivery device handle, where they can be digitized and processed by FPGA 153, and communicated as needed to FPGA 152. Magnetic field sensors 161 (e.g., Hall sensors) can be configured to sense the magnetic field of the solenoid 156, from which the position of the magnet relative to the retracted position can be computed by FPGA 153 and relayed to FPGA 152 for real time feedback of solenoid current to control magnet position.

Switch states for the activation of vapor and saline flush and control of needle movements can be sensed by FPGA 153 and communicated to the console and FPGA 152 for activation of these functions.

The console of FIG. 1E can further include a microprocessor 163 (MCU) that may communicate with both FPGA 152 and a single board computer 164 (SBC) that controls the graphical user interface 165 (GUI). In one embodiment the MCU communicates with one-wire security chips that typically reside near a cable connector, which may be a cable connecting the cartridge to the handle and/or the cable connecting the handle to the console. The MCU reads security chip information such as serial number, therapy mode, and information about prior uses, and writes information such as number of vapor shots and parameters of each shot.

Accessory sensory inputs and coil driver outputs may be connected through the console of FIG. 1E. For example, the tracking coils 162 shown in FIG. 1E may function as electromagnetic field sensors or as current drivers for generating AC magnetic fields. Needle tip tracking data may be computed in the console SBC and displayed to the user on the GUI or on external monitors. Other data that can be input to the console include real time images such as ultrasound or cystoscope images and sensing and control of saline irrigation to tissues surrounding the prostate to keep them cool during vapor delivery.

It should be understood that electronic components shown in FIG. 1E can communicate with some or all the other illustrated electronic components.

**Real-time vapor delivery needle tracking**

The vapor delivery needle of the vapor delivery system is configured to be advanced through the prostatic urethra into the prostate for vapor delivery. However, care must be taken to ensure that the needle does not advance completely through the prostate and puncture the prostatic capsule, which could result in vapor being delivered accidentally outside of the prostate into the patient's body. Described herein are systems, techniques, and methods for precision tracking of the location of the vapor delivery needle, and particularly the vapor delivery needle tip, to ensure that the vapor delivery needle does not accidentally puncture or advance through the prostatic capsule.

*Trans-rectal ultrasound imaging*

In some embodiments, a trans-rectal ultrasound imaging system (TRUS) is used in an adaptive mode to keep the plane containing the needle tip in focus. The plane of the needle tip is kept in focus by adjusting the ultrasound viewing angles. Mechanisms that translate and rotate the imaging element facilitate auto-focusing. In some embodiments, sensors or transmitters can be placed on the TRUS to track the location of the needle tip relative to the ultrasound image. In other embodiments both the needle tip and TRUS locations are tracked and superimposed. In this case, sensors may be placed on the TRUS to track the probe location with an array of sensors that are also tracking needle tip movements.

In one embodiment, the vapor delivery needle tip can be imaged by a TRUS probe. The vapor delivery needle tip can be visualized in real-time on the ultrasound image when it lies in the imaging plane of the TRUS transducers. The image of the vapor delivery needle may be enhanced with an ultrasound contrast material (for example a porous material) placed at or near the needle tip. In other embodiments reflective materials placed at the needle tip may enhance tip visibility. For example, features on the electrode tip such as gold-plated tip electrodes or copper GPS coil(s) may increase reflectivity and visibility of the needle tip. Additionally, the vapor delivery needle tip may be clearly identified by releasing a puff of steam or vapor from the needle tip holes, since vapor is a good ultrasound contrast agent, and shows a bright cloud around the tip in the image. A Doppler ultrasound image can additionally show a map of vapor velocity, which is largest at the vapor exit holes at the needle tip. The ultrasound transducer head may be translated and/or rotated to focus in on the needle tip. In this approach the needle tip, at the centroid of vapor ablation, can be identified before, during and after vapor ablation. Movement of the needle to the next ablation site may be unambiguously identified on the ultrasound image by keeping the needle tip in focus during the movement. In some embodiments, a computer controller can be configured to identify the needle tip in the ultrasound image, and automatically adjust the translation and rotation of the TRUS probe to keep the tip image in focus during needle movements.

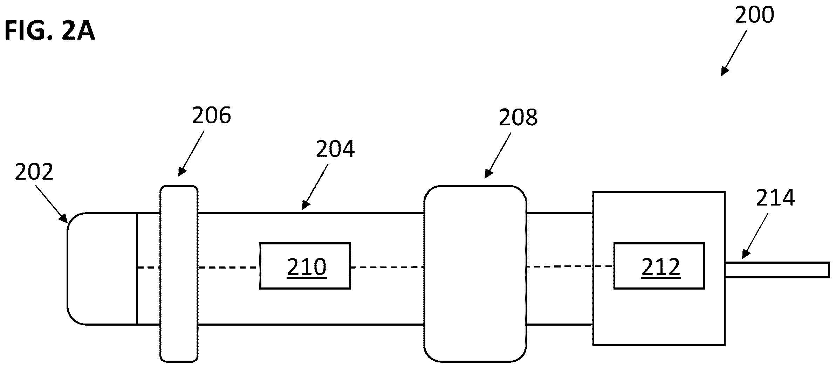

One embodiment of a TRUS probe 200 with auto focus is shown in FIG. 2A, which includes transducer 202, an immobile sheath 204, an inflatable stabilization balloon 206 located at a distal portion of the TRUS probe, an inflatable cuff balloon 208 located at a proximal portion of the TRUS probe, a translation/rotation actuator 210, electronics 212, and an optional battery (for wireless operation), and an optional flexible cable 214 (for wired operation). As shown, the ultrasound transducer head of the TRUS probe can be rotated and translated within a rigid, immobile sheath by a miniature, computer controlled linear and rotational actuator 210, controlled by electronics 212. Power and data can be carried by a flexible cable to a control computer. In some examples, power is provided by a battery contained within the probe, and data is communicated by a wireless transceiver which eliminates the need for the flexible cable.

The TRUS probe can be inserted into the patient's rectum and be placed proximate the patient's prostate. Once in position, the TRUS probe may be stabilized by inflating the distal stabilization balloon 206. If a gas is used to inflate the balloon, no portion of the balloon should lie between the ultrasound transducer and the prostate tissue being imaged, as gas attenuates and scatters the ultrasound beam. If any part of the balloon lies between the transducer and prostate tissue, the balloon should be inflated with a fluid that does not distort the ultrasound image, such as degassed water or saline. An inflatable cuff balloon 208 can be inflated to further stabilize the TRUS probe. The system controller may automatically maintain focus on the vapor delivery needle tip using the combined electromagnetic tip tracking and ultrasound imaging system presented below, or the user may adjust the focus via a user interface.

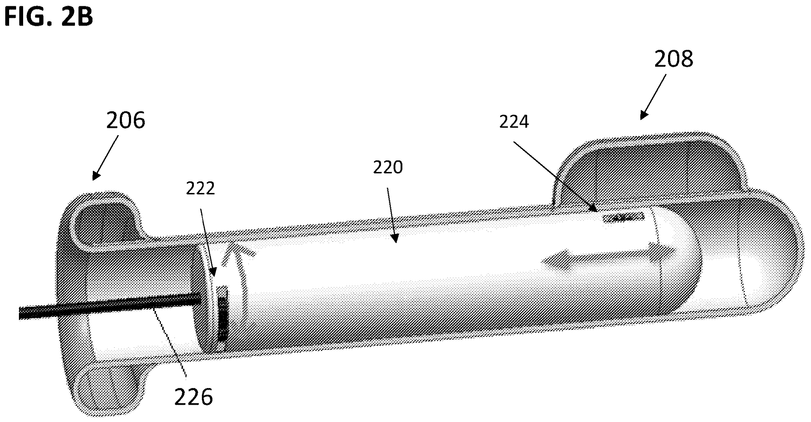

FIG. 2B is a cross sectional view which shows the internals of the TRUS probe, including the features that allow for translation and rotation of the TRUS transducer 202 within the balloon stabilized sheath. The TRUS transducer can include a pair of stepper motors 222 and 224 configured to engage a central rod or cable 226. The stepper motor 222 can be configured to rotate/translate the transducer 220 radially about the rod or cable 226, and the stepper motor 224 can be configured to advance/retract the transducer linearly along the rod or cable 226.

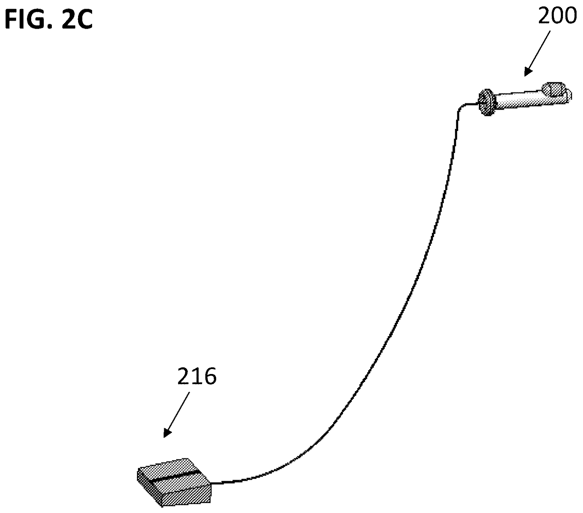

FIG. 2C shows an embodiment in which a physician or user of the system can activate translation and/or rotation of the TRUS transducer 200 with a foot pedal 216 to keep a portion of the ultrasound image, for example the needle tip, in focus during the procedure.

*Methods for vapor therapy guidance*

Methods for safe and effective vapor delivery to the prostate include avoiding prolonged or excessive vapor delivery to tissues near the prostate capsule to avoid damage to tissues on the outside of the capsule, including nerves and tissues of the rectum. Vapor is not delivered within a critical distance from the prostate capsule. Short vapor delivery shots with ample time for tissue cooling between shots, prevents excessive conduction of heat outside the prostate capsule.

Vapor therapy may be delivered focally to an area within the prostate that is known to be cancerous through MRI image analysis and/or tissue biopsies. Alternatively, a specific zone of the prostate or one hemispherical side of the prostate gland may be ablated in a single vapory therapy delivery session. In some procedures the entire prostate gland may be ablated using vapor therapy. In all these examples, it is important to deliver vapor to selected target locations within the prostate. A single vapor therapy shot may ablate tissue in a roughly spherical region having a diameter of one to two centimeters depending upon the power and duration of therapy delivery. Vapor may be reflected from the prostate capsule or from tissue boundaries between prostate zones. In these cases, the lesions may not be spherical, and may follow a path through the tissue of a specific prostate zone. Peripheral zones of the prostate, where prostate cancer primarily originates, may comprise thin layers adjacent the prostate capsule that extend over large portions of the prostate. The goal of any cancer therapy is to ablate all cancer while minimizing damage to non-cancerous tissue.

For these reasons, it is important to know where vapor therapy is being delivered within the prostate, and to assess the ablated tissues so that the location of subsequent vapor delivery can be planned. As mentioned above, vapor delivered from a vapor delivery needle can be seen on an ultrasound image as bright reflections from the vapor bubbles and/or from Doppler images of the speed of the vapor. Ablated tissue may be indicated on ultrasound images taken after vapor therapy as regions that appear darker due to the presence of condensed vapor. Digital subtraction images that show a map of differences before and after therapy, may enhance the contrast of ablated tissue.

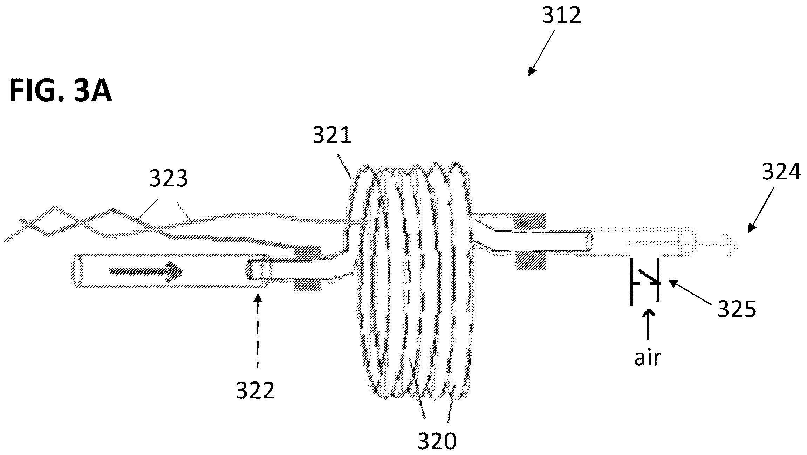

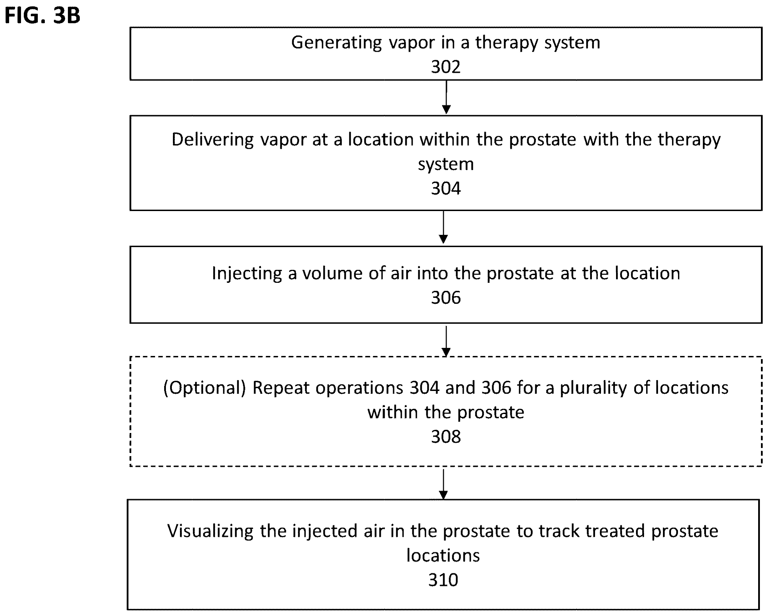

In one embodiment, the vapor generator of the system can be configured to introduce air into the vapor stream. Referring to FIG. 3, vapor generator 312 can include coil 320 comprising a plurality of loops of an insulated tubing 321, configured to receive fluid such as sterile water via an inlet 322. Current can be applied to the coil via leads 323 to heat the coil and produce a vapor at outlet 324. In this embodiment, air can be introduced into the vapor stream by opening the electronic valve 325 to air and closing the electronic valve just prior to the end of the vapor delivery. Air is pulled into the vapor stream by the Venturi effect. In other embodiments, air can be injected into the vapor stream, for example, by using a pump or fan to inject or deliver air. In use, air remains in the ablated tissues after the vapor delivery. It is slowly resorbed over a period of time (e.g., over a period of minutes). Ultrasound images observed after vapor delivery will appear bright in zones of ablation where it is reflected from the air, thereby providing a map of ablated tissues. Such images may be saved and retrieved for later assessment.

Similarly, referring to FIG. 3B, a flowchart is provided illustrating a method for tracking prostate therapy. The method can be performed using any of the systems and devices described herein. Referring to operation 302, the method can include generating vapor in a therapy system, and at operation 304, delivering vapor at a location within the prostate with the therapy system. As described herein, a vapor delivery system can include a transurethral shaft and a vapor delivery needle configured to access the prostate via the prostatic urethra. During therapy, a user of the system can move the shaft to the desired position within the patient's urethra, and extend the vapor delivery needle out from the shaft and into the prostate.

At operation 306 of the flowchart, the method can include injecting a volume of air into the prostate at the location. This injection of air can occur prior to, during, or after the vapor is delivered into the location at operation 304. As described above, in one embodiment a vapor therapy system can include a vapor generator with an air inlet that allows for an injection of air into the vapor stream by opening/closing a valve. In some embodiments, the method can include injecting a known volume of air into the prostate at the location. In other embodiments, the user of the device can inject any volume of air into the location. For example, it may be desirable to inject a larger volume of air into the prostate at a first or last treatment location.

At an operation 308, the method can optionally include repeating the operations 304 and 306. Specifically, the method can include placing the vapor delivery needle at one or more additional locations within the prostate and delivering vapor at each of the locations. Additionally, the method can include injecting a volume of air at each of these additional locations. In some embodiments, the volume of air injected can be the same for each location. In other embodiments, more or less volume of air can be injected. For example, it may be desirable to inject a larger than normal volume of air for the first, or last, location. This can then be used later to determine where the prostate treatment began or ended.

At an operation 310 of the flowchart, the method can include visualizing the injected air in the prostate to track the treated prostate locations. In some embodiments, visualizing the injected air can comprise visualizing the prostate with an ultrasound imaging system. As described above, injected air in the prostate will appear on ultrasound imaging as a bright or white volume within the prostate tissue. Therefore, locations within the prostate that have been treated with vapor and received an air injection will be easily visible in ultrasound imaging. In some embodiments, the method can further include the step of creating a map of treated locations within the prostate. The map can then be displayed to a user of the system to provide real-time feedback of treatment progress.

*Needle tip tracking with delivery device shaft sensors*

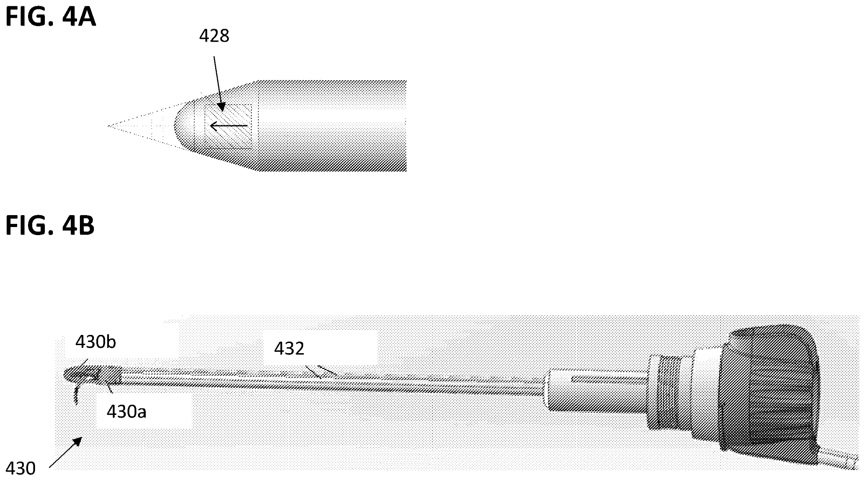

Methods and techniques for safe and effective navigation of the vapor delivery needle can include image guidance using real-time ultrasound and/or pre-operative MRI images. In one embodiment, referring to FIG. 4A, vapor delivery needle tip tracking can be facilitated by a magnet 428 embedded in or near the tip of the vapor delivery needle. While the illustrated embodiment shows a permanent magnet in the needle, it should be understood that this component could also be referred to a transmitter of magnetic field.

Additionally, referring to FIG. 4B, one or more sensors 430, such as magnetic sensors, can be placed at or near a distal end of the vapor delivery device shaft, and/or at strategic locations around the operating site. Sensor leads 432 along the shaft of the device can electrically couple the sensors 430 to the console or electronic controller, for example. The sensors 430 can comprise, for example, an axial winding 430a (e.g., a coil winding around the perimeter of the shaft) or side windings 430b (e.g., coil windings positioned on sides of the shaft). The position of the sensors can be fixed relative to where the vapor delivery needle exits the shaft.

In one method of use, navigation of the device can occur in pulse steps in which the vapor delivery needle tip moves a short, predetermined distance in a short time. Changes in vapor delivery tip location can be sensed during the pulse movements of the needle. For example, magnetic fields generated by the pulse movements of the magnet on the needle tip can be sensed by the sensors on the shaft of the device, and the value of the sensed magnetic field can be used to determine how far the tip of the vapor delivery needle is from the sensors, and therefore, how far the needle tip has advanced from the shaft of the vapor delivery device. In some embodiments, as described above, the distance that the needle has moved is known from measurements made by Hall sensors of the needle driver magnet position. In this case, sensors 430 on the shaft tip can indicate the lateral deflection of the needle from a nominal needle tip location along a line perpendicular to the delivery device shaft.

In the embodiment of FIG. 4B, the sensors 430 can comprise a plurality of orthogonal coils of wire located on or near the distal end of the shaft. In one embodiment, two coils can be wound on the sides of the shaft tip and a third can be wound around the axis of the shaft, as shown. Sensing the magnetic field of the needle tip magnet 428 as it moves provides a track of the x, y, and z coordinates of the tip relative to the shaft tip, relaxing the assumption that the needle path is known. The measured track of the needle tip may be at an angle to the shaft and may be curved, and may be registered onto a TRUS image, since the shaft is visible in the ultrasound image.

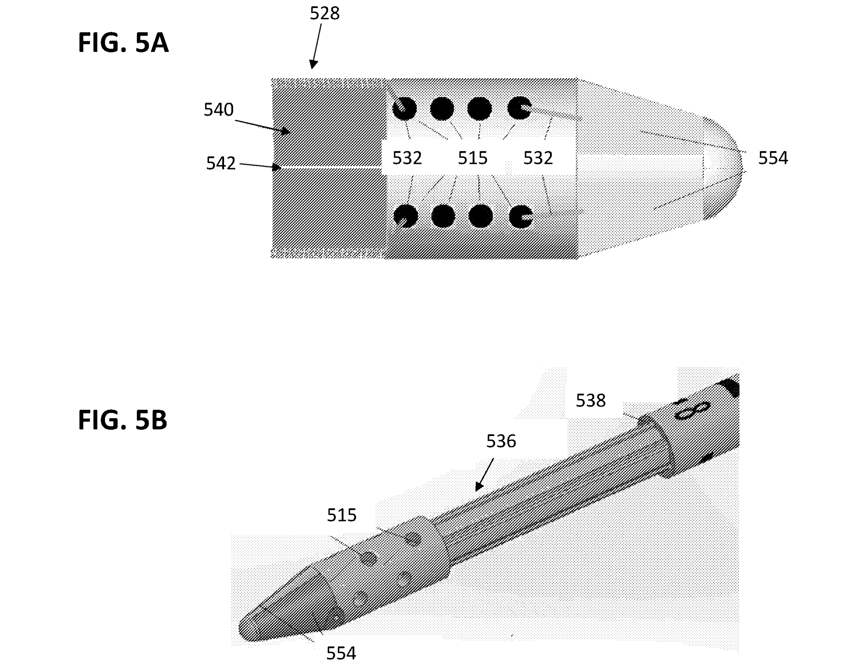

In some embodiments, the needle tip magnet 428 can be a coil of wire 528 positioned near the needle tip, as shown in FIGS. 5A. Referring to FIG. 5A, the coil can be positioned proximally from the needle tip, including the vapor delivery ports 515 and bio-impedance electrodes 554. As shown in FIG. 5A, in one embodiment the leads 532 for the bio-impedance electrodes and the coil 528 can be routed through the vapor delivery ports into the needle. However, in another embodiment, shown in FIG. 5B, the needle can include a slot 536 with channels 538 to accommodate the coil and leads, respectively. A sinusoidal current at a given frequency may be passed through the needle tip coil in FIGS. 5A-5B to create an alternating magnetic field that is sensed by the shaft tip sensors of FIG. 4B (e.g., the axial or side coil sensors 430). In one embodiment the shaft tip is never more than about 26 mm from the needle tip. In this case a relatively large signal with a very large signal to noise ratio is received by the sensors of FIG. 4B.

In one example, the shaft sensors of FIG. 4B can be coils of N = 100 turns of AWG #50 wire having a diameter of 0.001". In this example the coil radius is 4 mm and the coil area is $A = 5 \times 10^{-5}$ m². The coil has no core so the relative permeability $\mu = 1$. The voltage induced in the probe coils is equal to:

$$V = d\Phi/dt = 2\pi f \mu N A B \qquad (1)$$

At a frequency of 5,000 Hz, at the upper end of the frequency range typically used for magnetic tracking within the human body, Eq.(1) may be written

$$V/B = \text{sensitivity} = 160 \text{ Volts/Tesla} \qquad (2)$$

The side magnetic field at a separation r from the needle tip transmitter coil of Figure 6, considered as a magnetic dipole, is equal to:

$$B = m/(4\pi r^3) \qquad (3)$$

Where the magnetic moment m of the needle tip coil is:

$$m = \mu_0 \mu n a I \qquad (4)$$

In one preferred embodiment, the permeability, $\mu$, of the needle coil in FIG. 5A is equal to 3, the coil is wound with n = 300 turns of AWG #51 magnet wire, and $a = 1 \times 10^{-6}$ m² is the coil cross sectional area in the wall of a 1.25 mm vapor delivery needle. The current, I, is chosen as the maximum AC current at 5,000 Hz that can be delivered before the coil becomes hot to the touch. A typical safe current in this coil is 0.04 amps rms. The coil permeability can be provided by winding the coil over a permeable foil 540 or by winding the coil with a magnetically permeable wire, for example nickel magnet wire. In embodiments with a permeable foil, a gap 542 can be provided in the permeable foil to prevent circumferential eddy currents in the foil that tend to cancel the magnetic field generated by the coil. In this example, the magnetic moment of the needle tip coil in Eq.(4) is then m = 4.5 x $10^{-11}$ T-m$^3$, and the magnetic field of Eq.(3) at the maximum separation of 0.026 m is 2 x $10^{-7}$ Tesla rms, and the voltage induced in a shaft tip sensor from Eq.(2) is V = 32 x $10^{-6}$ Volts rms. A typical low noise amplifier has a noise voltage typically in the range of 3 x $10^{-9}$ x sqrt(BW) volts rms, where BW is the output signal bandwidth. If the measured rms voltage is averaged down to a rate of 10 samples per second, the bandwidth is 10 Hz, and the rms amplifier noise is about 9.5 x $10^{-9}$ volts rms. The expected signal to noise ratio at the largest separation between the needle tip and the shaft sensor is then:

$$S/N = 32 \text{ x } 10^{-6}/9.5 \text{ x } 10^{-9} = 3{,}400.$$

In other words, at the relatively small separation between the needle tip transmit coil and the shaft tip sensor the received signal is more than 3,000 times the noise. This can be translated into an expected uncertainty in the computed position of the transmitter coil by combining Eqs. (2) and (3) and differentiating to see that:

$$\delta r = r/(3S/N) = 0.0025 \text{ mm}$$

or negligible, and therefore sensor noise will not be the limiting factor in locating the needle tip relative to the shaft tip.

For the needle tip magnet of FIG. 4A the magnetic field of the stationary magnet is at DC, and it is well known that many noise sources, both natural and man-made make it difficult to discern a DC signal from such a small magnet. The induction coil shaft sensors of FIG. 4B have zero sensitivity at DC and an induced signal that increases with frequency. If the needle is moved rapidly in small steps at high speeds, the sensors of FIG. 4B will see an AC signal corresponding to the rise time of the step movement of the magnet. In the embodiment of FIG. 4A, the embedded permanent magnet has no leads with no electronics required for the needle tip. This is an advantage versus a needle tip coil because placing leads within the needle with no impact on other needle functions is challenging. As the magnet moves in pulse steps, the voltage induced in these coils is the time rate of change of the magnetic flux linking the coils:

$$V_{ind} = -d\Phi/dt = -(d\Phi/dx)(dx/dt) \tag{5}$$

Where $\Phi$ = magnetic flux in Tesla-m$^2$

dx/dt = magnet speed in m/sec
$V_{ind}$ = induced voltage in volts

To estimate the magnitude of the magnetic flux linking one of the coils, the magnet is considered a point dipole, having a minimum (side) field at a distance x from the needle tip of:

$$B = m/(4\pi x^3) \tag{6}$$

$$dB/dx = -3m/(4\pi x^4)$$

where

B = dipole magnetic field in Tesla
m = dipole moment of tip magnet in Tesla-m$^3$
x = distance between the tip magnet and sensor in meters

For oriented rare earth magnets, the dipole moment is given by:

$$m = MV \tag{7}$$

where M is the magnet magnetization and V is the magnet volume. The magnetization, M, is equal to the residual induction $B_r$ for these oriented magnets. A magnetic material is chosen that is resistant to the elevated temperatures at the tip during vapor delivery. For such magnets, the residual induction may be as low as M = 1.3 Tesla. Considering a cylindrical tip magnet of FIG. 4A having dimensions of 0.030" diameter by 0.030" long, the magnetic moment in Eq.(7) becomes:

$$m = 3.5 \times 10^{-10} \text{ Tesla-m}^3$$

The distance between the tip magnet and sensors located in the tip may be as much as x = 25 mm = 0.025m, and at this distance, Eq.(6) gives the magnetic field and its gradient:

$$B = 1.8 \times 10^{-6} \text{ Tesla} \qquad\qquad (8)$$

$$dB/dx = -2.1 \times 10^{-4} \text{ Tesla/m}$$

For a coil sensor, the magnetic flux linking the coil having N turns of area A is:

$$\Phi = NAB$$

$$d\Phi/dx = NAdB/dx$$

Given a coil having 100 turns of radius 4 mm,

$$\Phi = 9 \times 10^{-7} \text{ Tesla m}^2$$

$$d\Phi/dx = -1.1 \times 10^{-4} \text{ Tesla-m}$$

Equation (5) gives an estimate of the voltage induced in the sensor coil with an assumed tip speed of about 0.5 m/sec:

$$V_{ind} \approx 5.3 \times 10^{-5} \text{ volts}$$

A standard precision amplifier chip has input voltage noise of 3 nV/Hz$^{1/2}$. In a bandwidth of 100 Hz centered around an expected frequency (=1/pulse travel time) of 1000 Hz, the sensor noise is 30 nV or:

$$V_{noise} = 3 \times 10^{-8} \text{ volts}$$

So the minimum signal to noise ratio expected for localization of the needle tip sensor by shaft tip coil sensors is estimated as

$$\text{Signal/Noise} = V_{ind}/V_{noise} = 1,800$$

The accuracy of needle tip location is equal to the separation of the tip and shaft coil sensor divided by three times the signal to noise ratio. It is therefore expected that the coil tip sensors of this example will have a tracking accuracy of:

$$\delta x = x/5400 = 0.005 \text{ mm} \qquad\qquad (9)$$

which is negligible and shows that sensor noise will not be a factor in the accuracy of needle tip localization relative to the delivery device shaft. The user may perceive the needle tip movement as continuous, but on a microscopic level the motion is comprised of pulses. For example, the pulses may be fast and 0.1 - 0.3 mm each. The induction coils on the shaft tip track changes in needle magnetic field, or changes in magnet position relative to the shaft tip coils.

The location of the shaft tip may be observed on a trans-rectal ultrasound image. A point and click device may allow the user to indicate the shaft tip position on an ultrasound monitor, while the needle tip position relative to the shaft tip is input to the monitor and displayed.

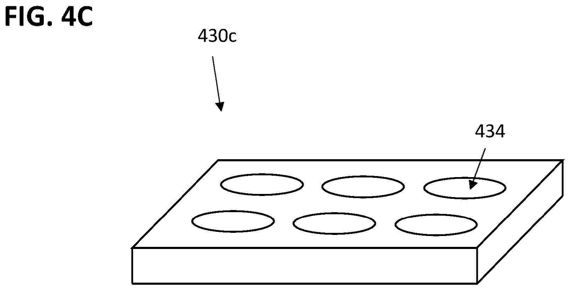

In another embodiment shown in FIG. 4C, the location and heading of the shaft tip is tracked using an external antenna array separate from the vapor device and vapor needle. In some embodiments, the external array can be placed external to the patient. For example, the external array can be integrated into a patient table or alternatively can be placed underneath or adjacent to the patient during treatment. The external array may comprise coils 434 that are transmit or receive coils. Conventionally, external tracking coils are transmitters that transmit sequentially or simultaneously at distinct frequencies, generally in the range of 1 - 5 kHz. The pulsed magnetic field from the tip sensor or tip magnet may be sensed at frequencies below 1 kHz and may be distinguished from the external signals by filtering. Alternatively, the external fields may be pulsed sequentially with the magnet movements to separate them in the time domain. The shaft tip location and heading is computed relative to the external array. This data may be transposed onto an image, such as an ultrasound image by placing magnetic sensors on the ultrasound probe and tracking coordinates of both the shaft tip and ultrasound probe. The tracking data may be transposed onto other images, for example a pre-operative MRI, using a registration process as discussed below.

In another embodiment, tracking sensors may comprise microchip magnet sensors, for example GMR, TMR, or AMR sensors. Requirements for sensor noise for these sensors may be computed from Eqs. (1) - (9) above. One advantage of magnetoresistive sensors is that their response goes down to DC. However, these sensors have 1/f noise, so the signal to noise ratio is lower at DC. In addition, background noise at DC can overwhelm the tip magnet signal. Pulsing the needle (and magnet) movement provides signals in a band near 1 kHz where sensor noise has reached its minimum value.

Both the solenoid coil currents and the solenoid magnet movement can be sensed by the shaft tip sensors as changes in the ambient magnet field and are thereby noise sources relative to the needle tip magnet or electromagnet. These fields are small due to the relatively large separation between the solenoid and the shaft tip and the rapid fall-off of the magnetic field with distance. These signatures are also known and measured by one or more sensors within the delivery device handle, and so they can be compensated. The displacement and speed of the needle deployment is obtained from delivery device sensors, which may be used to improve tip sensor tracking.

The number of leads required in the shaft and/or needle depends on the number of sensors. For example, a minimum of six leads are required for three sensors at the shaft tip. A plurality of leads entering the disposable delivery device cartridge requires a low-cost way of transferring signals from the cartridge to the reusable handle and/or the external system controller. Inexpensive wireless telemetry chips are available that can multiplex and digitize sensor signals and transmit a bit stream over a short distance with good resolution.

*Needle tip tracking with trans-rectal magnetic sensors or transmitters*

In one embodiment, as shown in FIG. 6A, the vapor delivery needle (such as the vapor delivery needle shown in FIGS. 5A-5B) can be tracked by a trans-rectal probe 600 that includes an array of magnetic sensors 630 disposed on or in the probe. In the illustrated embodiment, sensors 630 are shown near the distal end of the probe and also near the center of the probe between balloons 606 and 608. However it should be understood that the sensors can be placed anywhere on or in the probe. The magnetic sensors on the trans-rectal probe can be configured to sense the change in ambient magnetic field as the magnet or electromagnet on the vapor delivery needle tip is moved through the tissue. The electronics 612 of the probe can process the sensed signals to provide the x, y, z coordinates of the needle tip relative to the rectal probe. For the coil of FIG. 5A, Eqs. (2) and (3) may be evaluated with r equal to the largest separation anticipated between the rectal probe distal sensors and the needle tip. In the largest prostates at a needle location farthest from the rectal probe, the separation r will be less than 10 cm. The signal to noise ratio is equal to 60 at r = 100 mm, and the uncertainty in computed location is equal to 0.56 mm. The signal to noise ratio increases with the cube of the separation and the position uncertainty decreases with the fourth power of separation, so precision (sub-mm) tracking is realized at all points that the needle tip can access, in even the larges prostates.

In the embodiment of FIG. 6A, the trans-rectal probe including the magnetic sensor array can be inserted into the patient's rectum with the distal end of the probe positioned near the prostate gland. Balloons 606 and 608 may be inflated to stabilize the probe, as described above. However, unlike with trans-rectal ultrasound imaging, there is no requirement for good tissue contact with the probe of FIG. 6A, and minimal or no pressure needs to be applied to the probe. In contrast to TRUS, the prostate is not mechanically moved or altered by probe forces. Yet, the stabilizing balloons prevent movement of the magnetic sensor arrays, and a rigid framework for the sensors provides a rigid coordinate system for tracking the position of the vapor delivery needle.

Reference sensors 631 are separated from the sensors 630 that, when the probe is inserted into the patient, are proximate the prostate gland, and can be configured to provide measurements that may be subtracted from the primary tracking sensor measurements to compensate for environmental magnetic noise. This is especially important when DC field sensors are employed to sense a magnet in the needle tip. A microchip or electronics 212can be included within the trans-rectal probe to provide the x, y, and z coordinates and orientation angles of the needle tip.

The sensors in the trans-rectal probe may comprise multi-winding coils of fine wire, for example as shown by coils in FIG. 6B, or any solid-state magnetic field sensor that has adequate signal to noise ratio. Adding even more sensors provides redundant information that improves the tracking signal to noise ratio. Referring to FIG. 6B, the coils may include +y coil 630a, -y coil 630b, +x coil 630c, -x coil (not shown), +z coil 630d, and -z coil 630e. The trans rectal probe can be keyed 633 and hollow 635 to and adapted to receive an ultrasound probe. Coils 630a-e may be configured as transmitters when AC current is applied to the coils. A small coil on the vapor delivery needle can be configured to measure the AC magnetic fields of the coils 630a-630e to compute the location and orientation of the vapor delivery needle tip relative to the transmitter coils and therefore relative to the ultrasound image. An MRI image may be additionally be fused with the ultrasound image to show the needle tip track and current location on fused MRI/ultrasound images. In some procedures, the needle tip may be displayed on an ultrasound image without the MRI image or the tip may be displayed on a preoperative MRI image without employing ultrasound imaging.

In one embodiment shown in 6C, a pre-operative MRI 3-D image of the prostate can be displayed on a monitor. A stabilized rectal probe 600 with coil sensors 630 (such the sensors of FIG. 6B) collects data from the needle tip transmit coil 628 to provide tracking of the needle 606 that may be displayed or overlaid on the MRI image. In one embodiment shown in FIG. 6C, the ultrasound image, which is registered to the needle tip track, is also co-registered to the preoperative MRI image. The MRI image may show the location of cancerous tissue. With the needle retracted, the delivery device shaft may be tracked and advanced to a location and orientation within the urethra that is predicted to place a delivered needle into the cancerous region, where vapor can then be delivered.

Even without simultaneous display of the ultrasound and MRI images shown in FIG. 6C, the location of cancerous tissue may be manually input onto an ultrasound image if the coordinates of this tissue are known relative to the preoperative MRI image, and the coordinates of the MRI image are registered to the coordinates of the ultrasound image, for example by comparing coordinates of known landmarks in the two images.

Registration of the needle tip track to a preoperative MRI image is important to ensure the computed track of the needle tip is always near the true location of the needle tip as displayed on the MRI image. Because the MRI image is taken before the vapor therapy procedure, the location of some prostate tissue may have shifted. In addition, the delivery device shaft may alter the location of prostate tissues. Registration comprises electromagnetic determination of coordinates of anatomical features that appear on the MRI image.

In one registration process, the vapor therapy probe is moved to locations, as viewed on the cystoscope monitor, that can be identified on the MRI image, for example the bladder neck or verumontanum. In one example, the track determined by the rectal probe sense coils is scaled to make the track align with the MRI image of anatomical features. In another example, the MRI image is morphed to align with points determined by the electromagnetic track. In some embodiments the rectal probe sense coils are replaced by solid state or other magnetic field sensors. In some examples the coils on the rectal probe are energized with AC current to provide an alternating magnetic field that induces a voltage in a needle tip coil. The rectal coils may be energized sequentially, or at the same time if they are energized with different frequencies that can be resolved in the needle tip coil signal.

The needle tip coil (or magnet), especially when it is within the tip of the delivery device shaft and visible on the cystoscope can be used in another registration process. In an additional embodiment shown in FIG. 6D, a TRUS probe 601 can be used to register an MRI image to the patient's anatomy. The TRUS probe 601 can include ultrasound transducers 602, coils 603, leads 605a and 605b for the transducers and coils, and an optional protective sheath 606 (e.g., a condom, etc.). In some embodiments, the tool can include additional imaging capabilities such as a fiber optic camera. The probe can be moved within the rectum to locations that can be identified in the MRI image. These locations can be identified via ultrasound imaging or optionally via rectal probe fiber optic camera imaging. The probe coils or magnetic sensors 603 can be used for determining the coordinates of the visible anatomical feature. In some embodiments, the probe is steerable by actuating a lever on the device. Its small profile prevents mechanical disruption of the prostate tissue during the registration process. Electromagnetic localization can be facilitated by a set of external transmit coils, such as the external array shown in FIG. 4C. External transmit coils may be placed at other locations around the patient as needed to optimize tracking accuracy.

Other registration markers may be attached at points on the skin that lie over bony structures that appear on the MRI image, for example the hip bones and tail bone (coccyx). In some embodiments, coils of fine wire are adhesively secured to locations on the skin before the MRI procedure. The metal in the coil will appear in the image, especially if the coil is shorted. If these markers are left in place until the time of vapor therapy, they may be located by an external or internal electromagnetic (EM) tracking system. Since the markers appear on the MRI image, either the EM coordinate scaling or the MRI map may be altered in such a way that the markers overlap on the computed EM location and on the MRI image. This process may be repeated if the patient moves or the position of equipment such as the delivery device shaft changes enough to significantly alter the location of prostate tissues.

In the example of FIG. 6D, the coils 603 are wound on the probe to transmit AC magnetic fields at unique frequencies. Since the frequencies used are unique, the sensor outputs may be tightly filtered at these frequencies to increase signal to noise ratio. Driving sine wave currents into small coils at preferred locations on the probe is adequate to locate the probe, and in particular, the plane of the TRUS image relative to the delivery device needle tip. Coil leads are required on the TRUS probe that may plug into the external console of FIG. 1E.

A clinical goal is to track the path of the needle tip and display the track on an image of the prostate, for example a TRUS image that may or may not be registered to an MR image. In one embodiment, inertial sensor chips comprising accelerometers and solid-state gyroscopes can be mounted in the vapor delivery device. Translations along the axis of the delivery device shaft and rotations about the axis of the shaft can be measured by the inertial sensor chips. Registration to imaging, such as MRI imaging or ultrasound imaging, can be performed by identification of prostate landmarks using the delivery device cystoscope. The bladder neck and the verumontanum are outstanding landmarks that may be unambiguously identified. Rotation of the delivery device in the urethra can then be measured by the inertial sensor chips. A magnetic sensor placed in the delivery device handle is configured to measure the magnetic field of the solenoid needle drive magnet described above. Sensor measurements can be converted to the position of the magnet within the delivery device cartridge. In this way, the length of needle that has gone into the prostate is known. It may be assumed that the needle travels through prostate tissue in a straight line perpendicular to (or at a known angle to) the delivery device shaft, or on a curved but predictable path. With this assumption and the inertial sensor outputs, the location of the needle tip may be estimated and registered to the imaging. In other embodiments, a magnetic field sensor may be attached to the cartridge 104 of FIG. 1B. Magnetic fields transmitted from external antennas such as that of FIG. 4C can be used to locate the cartridge and thus the delivery device shaft tip relative to the external reference frame. One or more cartridge sensors will also provide the orientation of the cartridge and needle tip.

*Remote sensors for leadless needle tip tracking*

While multi-turn coils that may have a permeable core can achieve the highest sensitivity to magnetic fields, an array of flux-gate or solid-state magnetic sensors placed near the patient can also be used to measure the pulsed movements of the vapor delivery needle tip and localize five degrees of freedom of the needle tip that are the x, y, z coordinates and polar and azimuth angles of the tip relative to the sensor coordinate system. Typical rms magnetic field noise in a 100 Hz bandwidth centered at 1 kHz is $6 \times 10^{-10}$ Tesla. The side magnetic field from the tip magnet at 25 mm given in Eq.(3) may be extrapolated to another distance, r. For example, at 150 mm (about 6 inches) from the tip, the magnetic field is $8.3 \times 10^{-9}$ Tesla, which may be resolved with a signal to noise ratio of 83/6 = 14 using commercially available sensors, for example Honeywell HMC1003 sensors. In one example, two tri-axial sensors can be spaced apart on a rigid non-magnetic base and placed on or near the patient's lower torso. For example, the external coil array of FIG. 4C can comprise two or more tri-axial solid-state magnetic sensors. The two sensors supply six measurements of the tip magnet magnetic field, more than adequate to solve for the magnet's five degrees of freedom. Needle tip tracking is thereby achieved with a passive magnet component embedded into the needle tip and no leads at all on the needle or the delivery device probe. A variety of other magnetic sensors technologies, for example flux-gate and saturable core sensors, may be employed for sensing the moving permanent magnet or an AC magnetic field generated in a needle tip coil.

*Bio-impedance tissue sensing*

FIG. 5B also shows an improved vapor delivery needle tip, which includes needle tip bio-impedance electrodes 554 configured to measure the impedance of tissues surrounding the tip. Furthermore, the bio-impedance electrodes of the vapor delivery system can be used to distinguish between tissues of the prostate, for example, between the prostate tissues and the fibrous capsule tissues of the prostate. By sensing the resistance and capacitance of the tissue, the needle tip electrodes can be configured to sense an impedance change if/when the tip touches the wall of the prostate capsule. Impedance is measured by passing a constant current amplitude sine wave at a fixed frequency between the tip electrodes and measuring the voltage amplitude. The impedance amplitude is the ratio of voltage and current amplitudes. The phase shift between the voltage and current, along with the impedance amplitude is used to compute the tissue capacitance and resistance. The electrical resistance of the fibrous capsule tissue in the prostate is larger than the resistance of prostate tissue. The capacitance derives from the cellular tissue membranes. The less cellular capsule has smaller capacitance than prostate tissue, or a larger capacitive reactance. Both the increase in resistance of the capsule and decreased capacitance lead to an increase in impedance magnitude.

In one example, advancement of the vapor delivery needle can be automatically stopped if the electrodes sense an impedance rise that indicates that the needle is in contact with the prostatic capsule.

Both the resistance and capacitance within prostate tissue can change abruptly by up to 40% when the needle tip contacts the prostate capsule from inside the prostate. The contrast is largest in the frequency range between about 15 - 30 kHz. The range of frequencies that may be considered is between about 1 kHz and 10 MHz. At some frequencies a change in impedance can be seen in tissue after ablation therapy. Impedance may be different in cancerous and non-cancerous tissues. Therefore, in one embodiment, the bio-impedance measured at the needle tip can be used by the system to both detect the presence of cancerous tissue and to assess the success of vapor therapy in ablating the tissue.

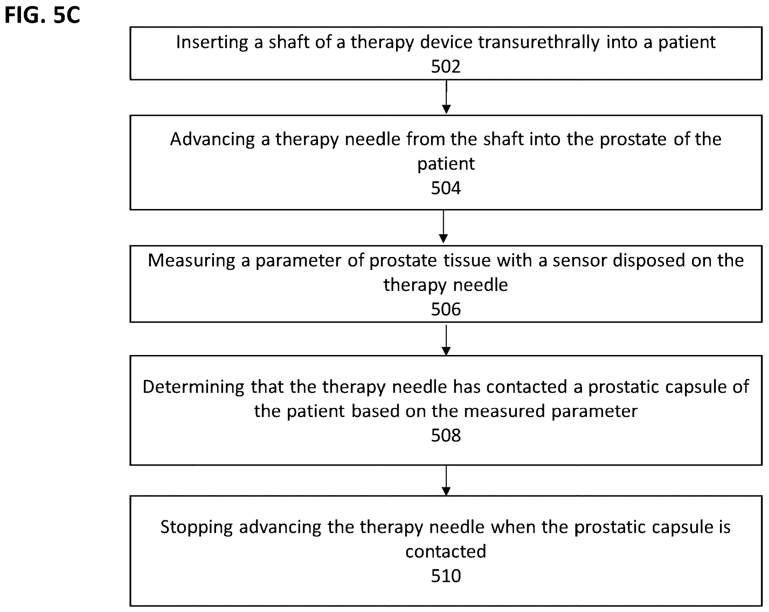

FIG. 5C is a flowchart providing one method of treating a prostate of a patient. The method can be performed with any of the systems or devices described herein. At an operation 502, the method of FIG. 5C can include inserting a shaft of a therapy device transurethrally into a patient. At operation 504, the method can further include advancing a therapy needle from the shaft into the prostate of the patient.

Next, at an operation 506, the method can further include measuring a parameter of the prostate tissue with a sensor disposed on the therapy needle. In some embodiments, the sensor can comprise a bio-impedance sensor and the measured parameter can be an electrical impedance of the prostate tissue. In other embodiments, other electrical parameters of the tissue can be measured by the sensor, such as electrical resistance or electrical capacitance. In yet another embodiment, the sensor can comprise a force sensor and the parameter can be a force applied by the prostate tissue against the needle tip. The parameter of the tissue can be continuously or periodically monitored as the needle is advanced into the prostate.

Next, at an operation 508, the method can include determining that the therapy needle has contacted a prostatic capsule of the patient based on the measured parameter. In one implementation when electrical impedance is being measured, the method can include determining that the needle has contacted the prostatic capsule of the patient if there is an abrupt change in the measured impedance. In some implementations, this abrupt change can comprise a change of more than 25% in the measured impedance. In some implementations, the abrupt change can comprise a change of more than 25-40%, or 20%-50% in the measured impedance In another implementation, when the sensor comprises a force sensor, the determining step can comprise determining that the needle has contacted the prostatic capsule when a force is applied to the needle that is consistent with the needle contacting the prostatic capsule at needle advancement speeds. This "critical force" can be known to the system, for example.

In some embodiments, the method can include providing an alert to a user that the prostatic capsule has been contacted by the needle tip. The alert can be, for example, a visual alert or an audible alert or alarm.

Next, at an operation 510, the method can include stopping advancing the therapy needle when the prostatic capsule is contacted. In some implementations, the needle advancement is stopped automatically. For example, a controller of the system can detect the contact with the prostatic capsule and automatically stop advancement of the needle. For example, as described above, a vapor delivery system can include a solenoid needle advancement mechanism. In one implementation, the electronic controller of the system is operatively coupled to the sensor and the advancement mechanism to automatically stop needle advancement when the system detects needle contact with the prostatic capsule.

*Tracking coils integrated with a TRUS probe*

As described above, the embodiment of FIG. 6D includes a TRUS probe 601in which electromagnetic coils 603 are integrated with a TRUS ultrasound imaging probe. The coils may be either transmitters or receivers, with the needle tip coil of the vapor delivery device being either a receiver or transmitter. In the former, the TRUS coils 603 can be driven by a sine wave current having a frequency below about 10 kHz. The low frequency limitation prevents significant induction of currents in conductive body tissues. In one embodiment, the coils can be driven at prime number frequencies that cannot be a harmonic of a lower frequency. The TRUS transmit coils may be driven sequentially at a single frequency or simultaneously at distinct frequencies. In one example the coils are driven sequentially at a frequency of 5 kHz. If localization data is desired at a 10 Hz rate, all data must be collected in 0.1 sec time intervals, so each coil can be on for a maximum time of 0.1/6 = 16.7 msec. Allowing 1.7 msec blanking time between coil excitations, each coil is ON for 15 msec. The time for one cycle of the sine wave is 1/5 msec = 0.2 msec. The number of cycles that each coil is on then 15/0.2 = 75, so data is averaged over 75 cycles, resulting in a noise reduction of square root of 75 or 8.7. In this approach there is a single sine wave current generator that is multiplexed to each of the six coils. The needle tip coil sensor signal can be conditioned via amplification and filtering at the coil drive frequency, and the output is collected in segments synchronized with the coil multiplexer.

Calculations using Eqs. (1) - (4) may be used to compute the signal to noise ratio and expected localization accuracy in the system of FIG. 6D. The coil current in the transmit coils is limited to a value that cannot damage rectal tissue by Ohmic $I^2R$ heating. In an example, the heating power is limited to 1 Watt. The coils are wound with about 150 turns of #42 magnet wire having a resistance of about 70 Ohms, so the critical current that dissipates 1 Watt of power in the coils is 0.12 Amps rms. Localization accuracy in needle tip coils is again sub-mm.

Needle tip coils may be wound with magnet wire in the range of #48 to #58 and a foil of high permeability and low hysteresis may be placed beneath the coil to magnify the needle tip coil signal by a factor of 2 to 10. As illustrated in FIG. 5A, a 10 micron thick single layer foil of Alloy 48, can be placed under the needle tip sense coil 528. In this embodiment, the sensed signal can be magnified by a factor of about 3. It is important in this regard to leave a small gap along the foil length to avoid induction of circumferential currents in foil that could cancel the signal. The added signal to noise provides an opportunity to reduce the area of the transmit coils. A disadvantage to using a permeable foil under the sense coil is that the foil may saturate in the relatively large magnetic fields found near to the transmit coils. An option to winding a copper coil over a permeable foil is to wind the coil with nickel or other magnetically permeable wire. As a sensor, the coil is connected to a high impedance amplifier, so very little current flows in the coil that could reduce the sensed voltage.

The transmit coils of FIG. 6D may be wound with magnet wire that provides sufficient flexibility to the coils, and may be driven by AC current and voltage levels that are safe and economically provided. In some embodiments, magnet wire in the range of #42 to #52 may be used to wind the transmit coils of FIG. 6D. The transmit coils may comprise any shapes that may be wound conveniently, including circles, ovals, squares, rectangles and even irregular shapes that can be quantified to compute magnetic fields using the law of Biot and Savart.

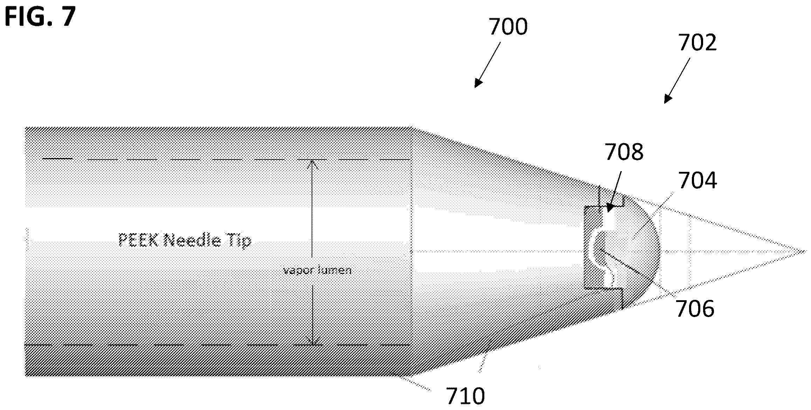

In some embodiments, the transmit coils may be integrated into the wall of a trans rectal tube as shown in FIG. 7B. In other examples, the ultrasound probe is inserted into the tube of FIG. 6B that comprises the electromagnetic field windings. The outside diameter of the tube may be in the range of 2.5 - 3 cm, and the inside diameter may be in the range of 2.3 - 2.8 cm. An ultrasound probe can be inserted into the transmitter coil tube in a keyed fashion that prevents the probe from translating or rotating with respect to the transmitter coils. In some embodiments the ultrasound wavelength is larger than the tube wall thickness, and the tube and integrated coils are essentially transparent to the ultrasound. In other embodiments, for example in FIG. 6D, the coils can be wound in a pattern such that the ultrasound beam does not intersect the wire of a coil. The tube material may be chosen so that the speed of the ultrasound wave is approximately the same in the tube material as in body tissues. Ultrasound gel, which has this acoustic impedance matching property, may be applied to the outside of the tube and inside the tube between the tube inside diameter and the ultrasound probe.

In some embodiments of FIG. 6B, the inside diameter of the tube is keyed to a trans-rectal ultrasound probe that is inserted into the transmitter tube. Locking the transmitter coils to the ultrasound probe guarantees that the ultrasound image is registered to the sensor tracking coordinate system, even when the TRUS probe is moved relative to the patient anatomy. In other words, after a factory calibration, the coordinate system of the magnetic field tracking system is the same as the coordinate system of the ultrasound image. This co-registration is an advantage over placing magnetic field transmitter coils outside the patient or at other locations that do not move with the ultrasound probe. A user may routinely translate or rotate the ultrasound probe within the patient's anus to find a best viewing angle for a given procedure. Without physically locked probes, such movements would need to be tracked and accounted for in an algorithm, or the tracking and ultrasound systems would need to be re-registered after each movement of the ultrasound probe. In some embodiments of FIG. 6D, the electromagnetic coils may be contained within thin wall of a flexible material that is impedance matched to the ultrasound frequency. The coils can be wound into the walls of a "condom" or protective sheath that may be rolled down onto the ultrasound probe after applying ultrasound gel to the probe. In this embodiment the condom must be placed onto the ultrasound probe so that the electromagnetic coils are in aligned properly with the TRUS transducer crystals to avoid coils crossing over the crystals and to provide a repeatable coil pattern on the probe.

One advantage of integrating electromagnetic localization with the TRUS or other rectal probe is that the transmit and receive coils are close to each other. As seen in Eq.(3), the magnetic field and therefore the magnitude of the coil sensor voltage, drops off as the cube of the separation between transmit and receive coils. Systems using external coils typically require more than 30 Watts of power to drive the transmit coils. In the examples cited above, sub-Watt power is needed for sub-mm localization accuracy. Low power reduces the size, cost and cooling requirements of electronics.

Another advantage of the trans-rectal coil system of this invention is that there is negligible interference of the transmitted signal due to external metals that may be in the operating area and that may change locations during a procedure. The transmitted signal is distorted by metal parts proximate the transmitter. No distortion is expected in the system of this invention because no metal objects are anticipated within or close to the prostate, and external metal objects are beyond the range of the system.

Another advantage of the trans-rectal coil system of this invention is that the sensor lead wires and electronics are exposed to negligible magnetic fields from the transmitter. In external transmit coil systems such as that of FIG. 4C, leads extending from the needle tip sensor and electronics in the delivery device handle of FIG. 1 may be exposed to magnetic fields that distort the received signal by inducing currents in the leads, connectors, and electronics components and ground planes.

Another advantage of the trans-rectal coil system of this invention is that the trans-rectal image is automatically registered to the EM track after calibration, and in contrast to external coil systems, registration cannot change during a procedure.

*Needle tip coil as a transmit coil*

In some embodiments the needle tip coil of FIG. 5A may be a transmitter of sine wave magnetic fields and the coil set in the rectal probe are receivers, as shown in FIG. 6D. Since the needle tip coil is truly much smaller than the separation between the sense and transmit coils, the dipole formula for magnetic field applies. Combining the equations for the transmit magnetic field and the sensed magnetic field yields the estimate of sensor voltage magnitude in the far field as:

$$V = \mu_t N_t A_t \mu_s N_s A_s (\mu_0 fI/r^3) \hspace{3cm} (12)$$

which is totally symmetrical in the product of permeability, number of turns, and area of the transmit and sense coils. The transmitter current is determined by the power dissipation allowed in the transmitter, which may be smaller for a needle tip transmitter, for example 1 Watt for the rectal probe transmitter and 0.1 Watts for the needle tip transmitter. The current and induced voltage signal scales with the square root of power, or in this example is reduced by a factor of SQRT(10) = 3.2. In this configuration the rectal probe coils can comprise more turns of finer wire. As a transmitter the resistance of these coils is limited to operate at a safe voltage, which limitation does not apply to the coil as a sensor. Advantages to using the needle tip coil as a transmitter versus a sensor include the use of a continuous sinusoidal transmit current with sensor voltages measured simultaneously, versus a transmit current that is multiplexed between multiple coils. Compared to sequentially activated rectal probe transmitter coils, the continuously operated rectal receive coils are sampled for a time longer by a factor of the number of coils, thereby improving the signal to noise ratio. It may be shown that the localization math is identical whether the measured voltages are induced in an array of external coils from a needle tip transmitter or the voltages induced in the needle tip coil are sensed for each external transmitter coil.

*General Needle Tip Tracking*

FIG. 6E is a flowchart providing one method of treating a prostate of a patient. The method can be performed with any of the systems or devices described herein. At an operation 62, the method of FIG. 6E can include inserting a shaft of a therapy device transurethrally into a patient. At operation 64, the method can further include advancing a therapy needle from the shaft into the prostate of the patient.

Next, at an operation 66, the method can further include determining a real-time position of the therapy needle in the prostate. In some implementations, determining the real-time position of the therapy needle can comprises determining the location of a sensor disposed on or in the therapy needle. For example, the needle can include a magnet or an electromagnet disposed on or in the needle. In some embodiments, a tracking system can detect the ambient magnetic field of the sensor as the needle moves through the prostate. The tracking system can comprise, for example, an array of transmitting coils. In some implementations, the tracking system can be integrated into the therapy device itself (e.g., on the shaft of the device), external to the device, or alternatively, can be integrated into a trans-rectal probe or a trans-rectal ultrasound imaging probe.

Next, at an operation 68, the method can include displaying the real-time position of the therapy needle and the prostate. In some implementations, this operation includes imaging the prostate with an ultrasound imaging system and displaying the image of the prostate with the location of the therapy needle overlaid onto the image of the prostate.

Next, at an operation 70, the method can include ablative therapy from the therapy needle to the prostate. As described herein, the therapy can comprise, for example, delivering vapor from the therapy system into one or more locations of the prostate to treat the prostate.

*Ultrasound imaging of vapor superimposed on MRI image*

Vapor is visible on ultrasound imaging as it exits the needle tip and spreads to adjacent tissue. In one embodiment, the needle tip location sensed by the tracking system may be superimposed onto the real time ultrasound image (which may be fused with an MRI image). The ultrasound monitor may display a predicted path of vapor into tissue around the needle tip. It can show the actual vapor spreading into tissue, and it may display previously ablated tissue. In some embodiments the tracking system is integrated with the ultrasound system and monitor. In other embodiments, a video combiner superimposes the output of the ultrasound monitor with tracking data from the EM system computer, and the combined image is displayed on a separate system monitor.

The user may view the ultrasound image with the superimposed track of the needle tip. An arrow may be placed on the current position of the needle tip to indicate its heading. Alternatively, the track of prior needle positions including the current position may itself be adequate indication of the needle heading. The user may observe the needle as it approaches the prostate capsule and stop needle advancement when the tip is close, for example about 5 mm, from touching the capsule. In another embodiment, the location of the needle tip relative to tissue such as the prostate capsule may be generated in an image processor that has been programmed to recognize the capsule in the ultrasound image. The vapor delivery device or system can be configured to alert the user and/or automatically stop needle movement when the vapor delivery needle approaches or touches the prostate capsule, thereby preventing puncture of the capsule with the potential for delivery of vapor to tissues outside of the prostate.

In some embodiments the delivery device handle can be attached to a robotic arm or platform that controls movements of the delivery device probe and needle tip, and automatically delivers the needle tip to locations specified by the user via a point and click or other input device. The robot may be programmed with user alarms or even automatic needle movements that prevent damage to tissues.

*Force or electrical contact sensors on needle tip*

A measurement of force acting on the tip of the vapor delivery needle may also be implemented in the systems described herein to alert the operator of needle contact with the prostate wall or capsule. A vapor delivery needle 700 having an electrical contact force sensor 702 is illustrated FIG. 7. In this example, the tip can be constructed from a flexible material 704 configured to flex a very small amount when the tip contacts the prostate wall or capsule. When a critical tip force is applied, electrical contact is made between hemispherical electrodes 706 and a contact switch 708 within the needle tip, sending an alert to the user via leads 710. The hemispherical shape of the electrodes compensates for off-axis contact of the needle tip with tissue. Tip material selection and the width, the nominal gap between tip electrodes, and tip geometry determine the force needed for electrical contact and user alert. Two leads can be used to run in a groove on the side of the needle or channels in the needle wall or in the needle vapor lumen to a microprocessor that determines contact has been made.

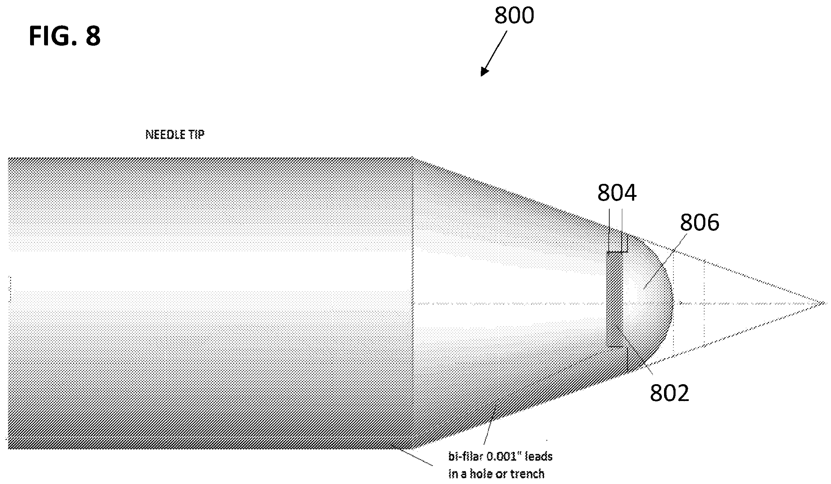

Another embodiment of a vapor delivery needle with a force sensor is shown in FIG. 8 in which a sensitive material 802 is sandwiched between two electrodes 804, with the outside electrode capable of flexing when a force is applied at the vapor delivery needle tip 806. Examples include a capacitor in which the material is a dielectric or even air or vacuum. The capacitance is measured and varies with electrode separation. Piezoelectric materials generate a voltage between the electrodes when force is applied. Piezoresistive materials change electrical resistance measured between the electrodes when force is applied. Sensor technologies include a magnetic field sensor in the tip that measures separation from a magnetic source such as the tip magnets described above. In another embodiment, the sensor may comprise a miniature integrated circuit that may contain micro-machined membranes or cantilevers, and may measure acceleration, displacement, or force. The minimum number of leads extending from a sensor at the needle tip, along a groove on the side of the needle, running through a channel in the walls of the delivery tube, or running through the needle vapor lumen, and exiting to electronics in the delivery device is two. If a Wheatstone bridge is integrated with the needle tip sensor, three leads are required. In some examples, fiber optics replace leads, and the sensor comprises a reflective membrane that flexes under needle tip force.

In the examples above, a critical force is detected, indicating that an obstruction such as the prostate capsule wall is impinging on the needle tip. The needle driver force is halted to immediately stop vapor delivery needle advancement, and the user can be referred to the ultrasound or MR image to check the location of the needle tip and alter its course to prevent penetration of the capsule.

As already discussed, the force exerted by tissue on the needle may be determined by measuring the current flowing in the solenoid coils 116 and 118 of FIG. 1A when the needle is being advanced through tissue at a constant speed. At constant speed, the net force on the needle is zero, that is, the force the tissue exerts on the needle is equal and opposite to the force that the solenoid exerts on the needle. Since the solenoid force is proportional to the solenoid coil current, measuring solenoid current is a measure of tissue force. An abrupt rise in solenoid current (the current needed to maintain constant speed) indicates that the needle tip has encountered an obstruction, which may be the prostate capsule.

As already discussed, a measurement of the electrical impedance of tissue lying between the electrodes of FIG. 5B can provide yet another indication of the approaching prostate capsule. It is worth noting that the electric field surrounding the needle tip extends to some extent in front of the needle tip. The voltage between the electrodes begins to rise somewhat before the needle tip encounters the capsule. This bio-impedance measurement provides an early warning of impending capsule tissue.

*Non-Newtonian needle tip material*

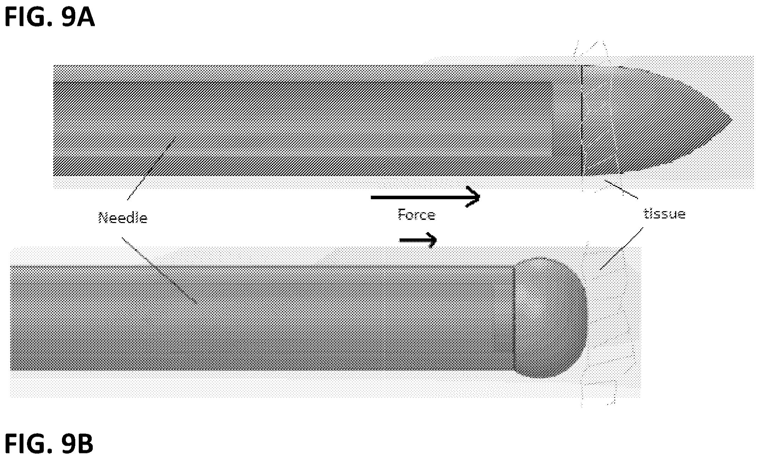

In one embodiment, the vapor delivery needle tip can be passively prevented from penetrating the prostate capsule wall by configuring the needle tip to flatten when contacting the prostate wall or capsule under the relatively small pulse navigation or continuous navigation forces, as shown in FIGS. 9A-9B. When the large and quickly acting needle deploy forces are applied, such as when the needle is initially advanced into the prostate, the needle retains its pointed shape to penetrate the urethral wall, as shown in FIG. 9A. However, when the needle is pressed slowly against a tissue with more rigidity such as the prostate capsule, the needle tip can be configured to flatten or blunt itself, as shown in FIG. 9B. In this embodiment, the material is referred to as non-Newtonian, a property of materials that remain rigid under large rapidly applied forces, but relax and deform under smaller, slowly applied forces. Wet beach sand is often cited as an example of such a material. Biocompatible materials such as PMMA beads are an example of a non-Newtonian material that may be used for the tip when surrounded by a shape memory polymer skin that returns the needle to its original shape when stress is relieved. PET is a candidate shape memory polymer.

*Shortened needle tip for vapor delivery to thin tissue zones of the prostate*

The peripheral zones of the prostate may lie adjacent the prostate capsule and can be thin relative to other zones of the prostate. When the vapor therapy needle is advanced perpendicular to the urethra, it may enter the peripheral zone parallel its thin dimension, which may be less than 10 mm thick. With the added length of the tapered needle tip, this product, when used to treat peripheral zone tissue, may accidentally treat adjacent zone tissue, may ablate the pseudocapsule separating prostate zones, or even penetrate the capsule. The vapor delivery needle described herein is designed to treat peripheral zone tissue, with the length of the needle segment having vapor delivery holes reduced to about 2.5 mm compared to 4 mm for the standard needle, as shown in FIG. 1D. The total area of holes may be made the same for the various tip designs, thus ensuring that the vapor velocity at the needle surface is about the same.

*Methods for safe and effective vapory therapy delivery*

A puncture resistant needle tip design and needle tip tracking superimposed on a real time ultrasound and/or MR image of the prostate mitigate the possibility of breaching the prostate capsule and delivering vapor to surrounding tissues. It is still possible for vapor to be conducted through the capsule wall to heat and injure tissues outside the prostate. Adjacent tissues of the rectum are susceptible to heat damage that can cause serious complications. Ejaculatory nerves are adjacent the outside walls of the prostate capsule, and thermal damage to these nerves may cause sexual dysfunction.

Safe and effective methods and systems for protecting sensitive tissues include limitation of heat conduction to these areas. Since heat conduction is a time dependent process, reducing the time that therapy is applied to tissues adjacent the prostate wall, and increasing the time for cooling between therapy shots are effective means for mitigating damage to periprostatic tissues. In a preferred embodiment, image guidance determines when the needle is adjacent sensitive tissues that need protection from overheating. Ablation of prostate tissue is performed in multiple steps that may comprise variable ablation times at a fixed calorie/second energy vapor delivery rate.

In some embodiments, normal saline is injected into tissues surrounding the prostate gland to provide a heat sink that prevents excessive elevation of temperature in tissues surrounding the prostate. Saline may be injected via one or more saline needles that are inserted into the prostate through the perineum. Saline injected proximate the prostate physically separates the prostate from surrounding tissue, creating a protective layer of saline. The saline also creates a fluid layer that is minimally reflective, or dark, on the ultrasound image, providing a sharp image of the prostate capsule. Unambiguous delineation of the prostate capsule along with needle tip tracking superimposed on the ultrasound image provides further protection against needle breach of the capsule. The 3-D outline of the prostate may be saved in the system computer and mathematically transformed and displayed on new views as the ultrasound imaging planes are changed during the procedure.

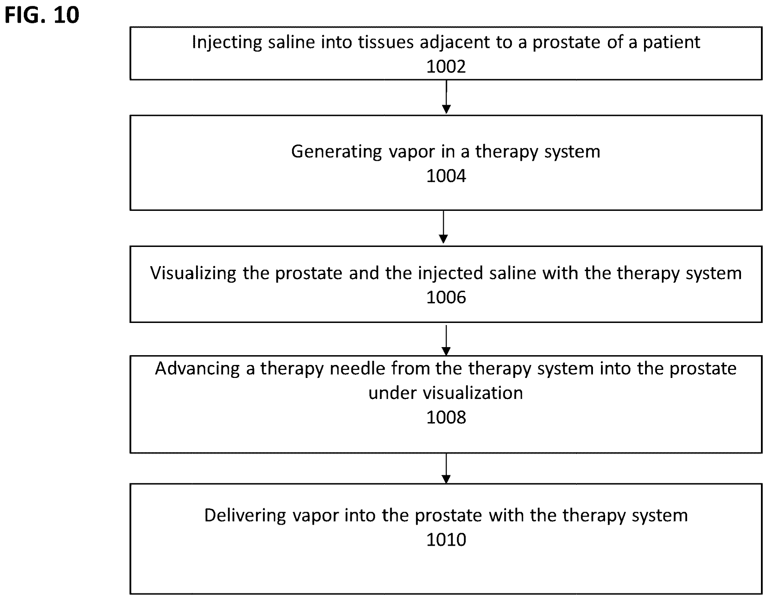

FIG. 10 provides a flowchart a method of treating a prostate. The method can be performed using any of the systems and devices described herein. At an operation 1002, the method can include injecting saline into tissues adjacent to a prostate of a patient. The saline can be injected, for example, with a needle inserted transperineally. In some implementations, enough saline can be injected into tissues adjacent to the prostate to completely surround the prostate. In one implementation, the saline injection creates a fluid layer between the prostate tissues and adjacent tissues.

Referring to operation 1004, the method can include generating vapor in a therapy system. At an operation 1006, the method can further include visualizing the prostate and the injected saline with the therapy system. In one implementation, the visualization comprises visualization with an ultrasound imaging system. In this implementation, the saline injection creates a fluid layer between the prostate and adjacent tissues that is visualized under ultrasound imaging as a dark or minimally reflective region in the patient.

At an operation 1008, the method can further include advancing a therapy needle from the therapy system into the prostate while under the visualization. In some implementations, the position of the therapy needle can be tracked and the dark or minimally reflective region provided by the injected saline can be used to ensure that needle advancement does not extend beyond the prostatic capsule into the adjacent tissues.

At an operation 1010, the method can further include delivering vapor into the prostate with the therapy system. As described herein, a vapor delivery system can include a transurethral shaft and a vapor delivery needle configured to access the prostate via the prostatic urethra. During therapy, a user of the system can move the shaft to the desired position within the patient's urethra, and extend the vapor delivery needle out from the shaft and into the prostate. Vapor can then be delivered into the prostate from the vapor delivery needle.

Although embodiments of the present invention have been described above in detail, it will be understood that this description is merely for purposes of illustration and the above description of the invention is not exhaustive. Specific features of the invention are shown in some drawings and not in others, and this is for convenience only and any feature may be combined with another in accordance with the invention. Variations and alternatives will be apparent to one having ordinary skills in the art. Such alternatives and variations are intended to be included within the scope of the claims. Features that are presented in dependent claims can be combined and fall within the scope of the invention. The invention also encompasses embodiments as if dependent claims were alternatively written in a multiple dependent claim format with reference to other independent claims.

The following are the claims of the parent application as filed and are included as part of the description of the present divisional application.

1. A prostate treatment device, comprising:

an introducer shaft sized and configured for transurethral access into a patient;

a therapy needle slidably disposed within the introducer shaft,

an advancement mechanism coupled to the therapy needle and configured to advance the therapy needle from the introducer shaft through a prostatic urethra into a prostate of the patient;

at least one sensor disposed on a distal portion of the therapy needle, the at least one sensor being configured to sense a parameter of one or more tissues of the prostate; and

an electronic controller operatively coupled to the at least one sensor, the electronic controller being configured to determine if the therapy needle contacts a prostatic capsule of the prostate based on the sensed parameter.

2. The device of claim 1, wherein the parameter comprises an electrical impedance of one or more tissues of the prostate.

3. The device of claim 2, wherein sensing the electrical impedance of the one or more tissues of the prostate further comprises:

passing a constant current amplitude sine wave at a fixed frequency to the at least one sensor;

measuring a voltage amplitude of the at least one sensor;

determining an impedance amplitude by calculating a ratio of the voltage amplitude and the current amplitude;

determining a phase shift between the voltage amplitude and the current amplitude; and

computing the electrical impedance of the one or more tissues with the phase shift and the impedance amplitude.

4. The device of claim 2, wherein the at least one sensor comprises at least one bio-impedance electrode.

5. The device of claim 2, wherein the controller is configured to determine that the therapy needle has contacted a prostatic capsule of the prostate when there is an abrupt change in the electrical impedance.

6. The device of claim 5, wherein the abrupt change comprises a sudden change of more than 25%.

7. The device of claim 1, wherein the parameter comprises an electrical resistance of one or more tissues of the prostate.

8 The device of claim 1, wherein the parameter comprises an electrical capacitance of one or more tissues of the prostate.

9. The device of claim 1, wherein the parameter comprises a force applied by one or more tissues of the prostate to the at least one sensor.

10. The device of claim 9, wherein the at least one sensor comprises a force sensor.

11. The device of claim 10, wherein the force sensor is embedded behind a flexible tip of the therapy needle, wherein the flexible tip is configured to flex when a critical force is applied to the flexible tip.

12. The device of claim 1, wherein the therapy needle is configured to deliver vapor into the prostate.

13. The device of claim 1, further comprising a magnet coupled to a proximal portion of the therapy needle, wherein the advancement mechanism comprises a solenoid actuator disposed around the magnet, the solenoid actuator comprising a push winding coupled to a source of current and a pull winding coupled to the source of current, the push winding being configured to apply a first magnetic field to the magnet, the pull winding being configured to apply a second magnetic field to the magnet, wherein the first and second magnetic fields move a distal tip of the therapy needle between a retracted position inside the introducer shaft and an extended position at least partially outside of the introducer shaft.

14. A method of treating a prostate of a patient, comprising the steps of:

inserting a shaft of a therapy device transurethrally into the patient;

advancing a therapy needle from the shaft, through a prostatic urethra of the patient, and into the prostate of the patient;

measuring at least one parameter of prostate tissue with a sensor disposed on the therapy needle;

determining that the therapy needle has contacted a prostatic capsule based on the at least one parameter; and stopping advancing the therapy needle when the prostatic capsule is contacted.

15. The method of claim 14, wherein the at least one parameter comprises an electrical impedance of the prostate tissue.

16. The method of claim 15, wherein determining that the therapy needle has contacted the prostatic capsule further comprises detecting an abrupt change in the measured electrical impedance.

17. The method of claim 16, wherein the abrupt change comprises a sudden change of more than 25% in the measured electrical impedance.

18. The method of claim 14, wherein the at least one parameter comprises a force applied by the prostate tissue to the therapy needle.

19. The method of claim 18, wherein determining that the therapy needle has contacted the prostatic capsule further comprises detecting a critical force with the sensor.

20. The method of claim 14, further comprising delivering vapor from the therapy needle into the prostate.

21. A prostate treatment system, comprising:

  a therapy device comprising:

    an introducer shaft sized and configured for transurethral access into a patient;
    a therapy needle slidably disposed within the introducer shaft;
    an advancement mechanism coupled to the therapy needle and configured to advance the therapy needle from the introducer shaft through a prostatic urethra into a prostate of the patient;

    at least one transmitter disposed on the therapy needle; and
    an external tracking system configured to sense a position of the at least one transmitter within the prostate.

22. The system of claim 21, wherein the at least one transmitter comprises a magnet, wherein the external tracking system is configured to sense a pulsed magnetic field from the magnet to determine the position of the at least one sensor within the prostate.

23. The system of claim 22, wherein the external tracking system comprises an array of transmitter coils configured to sense a change in ambient magnetic field of the magnet as it moves through the prostate.

24. The system of claim 21, wherein the external tracking system is disposed on or within a trans-rectal probe.

25. The system of claim 24, wherein the trans-rectal probe comprises a trans-rectal ultrasound probe.

26. The system of claim 22, wherein the magnet comprises an electromagnet.

27. A prostate treatment device, comprising:

  an introducer shaft sized and configured for transurethral access into a patient;
  a therapy needle slidably disposed within the introducer shaft;
  an advancement mechanism coupled to the therapy needle and configured to advance the therapy needle from the introducer shaft through a prostatic urethra into a prostate of the patient;
  at least one transmitter disposed on the therapy needle; and
  a tracking sensor disposed on a distal portion of the introducer shaft, the tracking sensor being configured to sense a position of the at least one transmitter on the therapy needle relative to the distal portion of the introducer shaft.

28. The system of claim 27, wherein the at least one transmitter comprises a magnet, wherein the tracking sensor is configured to sense a pulsed magnetic field from the magnet to determine the position of the at least one sensor.

29. The system of claim 28, wherein the external tracking system comprises an array of transmitter coils configured to sense a change in ambient magnetic field of the magnet as it moves through the prostate.

30. The system of claim 28, wherein the magnet comprises an electromagnet.

31. A method of treating a prostate of a patient, comprising the steps of:

inserting a shaft of a therapy device transurethrally into the patient;
advancing a therapy needle from the shaft, through a prostatic urethra of the patient, and into the prostate of the patient;
determining a real-time position of the therapy needle in the prostate;
displaying the real-time position of the therapy needle and the prostate; and
providing ablative therapy from the therapy needle to the prostate.

32. The method of claim 31, wherein the advancing step further comprises advancing the therapy needle and a transmitter disposed on the therapy needle into the prostate.

33. The method of claim 32, wherein determining the real-time position of the therapy needle further comprises sensing an ambient magnetic field of the transmitter with a tracking system.

34. The method of claim 33, further comprising sensing the ambient magnetic field of the transmitter with a tracking system disposed on the shaft of the therapy device.

35. The method of claim 33, further comprising sensing the ambient magnetic field of the transmitter with a tracking system external to the therapy device.

36. The method of claim 33, further comprising sensing the ambient magnetic field of the transmitter with a tracking system disposed on a trans-rectal probe.

37. The method of claim 33, further comprising sensing the ambient magnetic field of the transmitter with a tracking system disposed on a trans-rectal ultrasound probe.

38. The method of claim 37, further comprising registering the real-time position of the therapy needle onto an ultrasound image from the trans-rectal ultrasound probe.

39. The method of claim 31, wherein displaying the real-time position further comprises displaying the real-time position of the therapy needle within the prostate.

40. A method for tracking prostate therapy of a patient, comprising the steps of:

generating vapor in a vapor therapy system;
delivering vapor from the vapor therapy system into a first location within a prostate of the patient;
injecting a first volume of air from the vapor therapy system into the first location; and
visualizing the first volume of air in the prostate to track the prostate therapy.

41. The method of claim 40, wherein delivering vapor further comprises:

introducing a shaft of the vapor therapy system transurethrally into the patient;
advancing a vapor therapy needle from the shaft into the first location of the prostate.

42. The method of claim 40, further comprising:

delivering vapor from the vapor therapy system into a second location within the prostate;
injecting a second volume of air from the vapor therapy system into the second location; and
visualizing the second volume of air in the prostate to track the prostate therapy.

43. The method of claim 42, wherein the first volume of air is larger than the second volume of air.

44. The method of claim 42, wherein the first volume of air is smaller than the second volume of air.

45. The method of claim 42, further comprising creating a map of treated prostate locations.

46. The method of claim 40, wherein injecting the first volume of air is performed after delivering vapor into the first location.

47. The method of claim 40, wherein injecting the first volume of air and delivering vapor into the first location are performed simultaneously.

**Claims**

1. A prostate treatment device, comprising:

   an introducer shaft (102) sized and configured for transurethral access into a patient;
   a therapy needle (106) slidably disposed within the introducer shaft;
   an advancement mechanism (110) coupled to the therapy needle and configured to advance the therapy needle from the introducer shaft through a prostatic urethra into a prostate of the patient;
   at least one transmitter (428) of magnetic field disposed on the therapy needle; and
   at least one tracking sensor (430) disposed on a distal portion of the introducer shaft, the at least one tracking sensor being configured to sense a position of the at least one transmitter on the therapy needle relative to the distal portion of the introducer shaft.

2. The system of claim 1, wherein the at least one transmitter comprises a magnet.

3. The system of claim 2, wherein the tracking sensor is configured to sense the magnetic field from the magnet to determine a position of the therapy needle.

4. The system of claim 1, further comprising an array of sensors placed at strategic locations around the prostate configured to sense a change in ambient magnetic field of the sensor as it moves through the prostate.

5. The system of claim 2, wherein the magnet comprises an electromagnet.

6. The system of claim 5, wherein the therapy needle includes a slot (536) with channels (538) to accommodate the electromagnet and electrical leads (532) coupled to the electromagnet.

7. The system of claim 1, wherein the at least one tracking sensor comprises a coil winding around a perimeter of the introducer shaft.

8. The system of claim 1, wherein the at least one tracking sensor comprises a coil winding positioned on sides of the introducer shaft.

9. The system of claim 1, wherein the at least one tracking sensor is fixed relative to where the therapy needle exits the introducer shaft.

10. The system of claim 1, wherein the at least one tracking sensor comprises three orthogonal coils of wire to provide tracking of the therapy needle in x, y, and z coordinates relative to the introducer shaft.

11. The system of claim 1, wherein the advancement mechanism includes sensors configured to determine a distance the therapy needle has moved, wherein the at least one tracking sensor is configured to indicate a lateral deflection of the therapy needle from a nominal needle tip location along a line perpendicular to the introducer shaft.

12. The system of claim 1, further comprising a monitor configured to display a position of the therapy needle.

13. The system of claim 1, further comprising electronics configured to register a measured track of the therapy needle onto an ultrasound image of the prostate.

14. The system of claim 1, further comprising an external antenna array separate from the introducer shaft and therapy needle configured to track a location and heading of the therapy needle.

15. The system of claim 14, wherein the external antenna array comprises a plurality of coils (434) that are transmit or receive coils.

FIG. 1A

# Fig. 1B

EP 4 751 669 A2

**FIG. 1C**

# FIG. 1D

106

126

115

115

EP 4 751 669 A2

FIG. 1E

**FIG. 2A**

200

214

212

208

204

210

206

202

FIG. 2B

FIG. 2C

FIG. 3A

**FIG. 3B**

```
┌─────────────────────────────────────────────────────────────┐
│            Generating vapor in a therapy system               │
│                          302                                  │
└─────────────────────────────────────────────────────────────┘
                              │
                              ▼
┌─────────────────────────────────────────────────────────────┐
│   Delivering vapor at a location within the prostate with the │
│                      therapy system                           │
│                          304                                  │
└─────────────────────────────────────────────────────────────┘
                              │
                              ▼
┌─────────────────────────────────────────────────────────────┐
│   Injecting a volume of air into the prostate at the location │
│                          306                                  │
└─────────────────────────────────────────────────────────────┘
                              │
                              ▼
┌─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ┐
│  (Optional) Repeat operations 304 and 306 for a plurality of  │
│                  locations within the prostate                │
│                          308                                  │
└─ ─ ─ ─ ─ ─ ─ ─ ─ ─ ─ ─ ─ ─ ─ ─ ─ ─ ─ ─ ─ ─ ─ ─ ─ ─ ─ ─ ─ ─ ┘
                              │
                              ▼
┌─────────────────────────────────────────────────────────────┐
│  Visualizing the injected air in the prostate to track treated│
│                      prostate locations                       │
│                          310                                  │
└─────────────────────────────────────────────────────────────┘
```

# FIG. 4A

428

# FIG. 4B

430b

432

430a

430

EP 4 751 669 A2

**FIG. 4C**

430c

434

FIG. 5A

FIG. 5B

EP 4 751 669 A2

**FIG. 5C**

EP 4 751 669 A2

Inserting a shaft of a therapy device transurethrally into a patient
502

↓

Advancing a therapy needle from the shaft into the prostate of the
patient
504

↓

Measuring a parameter of prostate tissue with a sensor disposed on the
therapy needle
506

↓

Determining that the therapy needle has contacted a prostatic capsule of
the patient based on the measured parameter
508

↓

Stopping advancing the therapy needle when the prostatic capsule is
contacted
510

FIG. 6A

FIG. 6B

## FIG. 6C

628        cancer

Vapor delivery device

606

630

3-D MRI Image

600

EP 4 751 669 A2

**FIG. 6D**

**FIG. 6E**

Inserting a shaft of a therapy device transurethrally into a patient
62

Advancing a therapy needle from the shaft into the prostate of the patient
64

Determining a real-time position of the therapy needle in the prostate
66

Displaying the real-time position of the therapy needle and the prostate
68

Providing ablative therapy from the therapy needle to the prostate
70

EP 4 751 669 A2

FIG. 7

**FIG. 8**

800

NEEDLE TIP

804

806

802

bi-filar 0.001" leads
in a hole or trench

FIG. 9A

FIG. 9B

**FIG. 10**

```
┌────────────────────────────────────────────────────────────────┐
│   Injecting saline into tissues adjacent to a prostate of a patient │
│                              1002                               │
└────────────────────────────────────────────────────────────────┘
                                │
                                ▼
┌────────────────────────────────────────────────────────────────┐
│              Generating vapor in a therapy system               │
│                              1004                               │
└────────────────────────────────────────────────────────────────┘
                                │
                                ▼
┌────────────────────────────────────────────────────────────────┐
│  Visualizing the prostate and the injected saline with the therapy system │
│                              1006                               │
└────────────────────────────────────────────────────────────────┘
                                │
                                ▼
┌────────────────────────────────────────────────────────────────┐
│  Advancing a therapy needle from the therapy system into the prostate │
│                        under visualization                      │
│                              1008                               │
└────────────────────────────────────────────────────────────────┘
                                │
                                ▼
┌────────────────────────────────────────────────────────────────┐
│       Delivering vapor into the prostate with the therapy system │
│                              1010                               │
└────────────────────────────────────────────────────────────────┘
```

**REFERENCES CITED IN THE DESCRIPTION**

**Patent documents cited in the description**

- US 62955288